# EUROPÄISCHE PATENTSCHRIFT

(11) **EP 2 478 007 B1**
(45) Veröffentlichungstag und Bekanntmachung des Hinweises auf die Patenterteilung: **15.11.2017**
(21) Anmeldenummer: 10776278.3
(22) Anmeldetag: 15.09.2010
(51) Int. Cl.: C07K 14/415, C12N 15/82

(54) **INHIBIERUNG DES SCHOSSENS UND BLÜHENS EINER ZUCKERRÜBENPFLANZE**
INHIBITION OF BOLTING AND FLOWERING OF SUGAR BEET
INHIBITION DE LA MONTAISON ET DE LA FLORAISON D'UNE PLANTE DE BETTERAVE À SUCRE

(30) Priorität: 15.09.2009 DE 102009041333
(43) Veröffentlichungstag der Anmeldung: 25.07.2012
(73) Patentinhaber: KWS SAAT SE, 37574 Einbeck (DE)
(72) Erfinder: KRAUS, Josef, 37574 Einbeck (DE); MENZE, Andreas, 37077 Göttingen (DE); WURBS, David, 37574 Einbeck (DE)
(86) Internationale Anmeldenummer: PCT/DE2010/001081
(87) Internationale Veröffentlichungsnummer: WO 2011/032537

(56) Entgegenhaltungen:
- WO-A1-2007/122086
- WO-A1-2009/141446
- WO-A2-2010/025888
- SUNG SIBUM ET AL: "A PHD finger protein involved in both the vernalization and photoperiod pathways in Arabidopsis", GENES & DEVELOPMENT, Bd. 20, Nr. 23, Dezember 2006 (2006-12), Seiten 3244-3248, XP002616054, ISSN: 0890-9369
- JUNG C ET AL: "Flowering time control and applications in plant breeding", TRENDS IN PLANT SCIENCE, ELSEVIER SCIENCE, OXFORD, GB, Bd. 14, Nr. 10, 1. Oktober 2009 (2009-10-01), Seiten 563-573, XP026668259, ISSN: 1360-1385, DOI: DOI:10.1016/J.TPLANTS.2009.07.005 [gefunden am 2009-08-27]
- DATABASE EMBL [Online] 1. Januar 2008 (2008-01-01), "UFL_192_45 Cotton fiber 0-10 day post anthesis Gossypium hirsutum cDNA, mRNA sequence.", XP002616055, gefunden im EBI accession no. EMBL:ES815074 Database accession no. ES815074
- DATABASE Geneseq [Online] 18. Oktober 2007 (2007-10-18), "Glycine max cDNA SEQ ID NO:128994.", XP002616056, gefunden im EBI accession no. GSN:AFP37816 Database accession no. AFP37816 & US 2004/031072 A1 (LA ROSA THOMAS J [US] ET AL) 12. Februar 2004 (2004-02-12)
- DATABASE UniProt [Online] 24. März 2009 (2009-03-24), "SubName: Full=Putative uncharacterized protein;", XP002616057, gefunden im EBI accession no. UNIPROT:B9RPB3 Database accession no. B9RPB3

## Beschreibung

Die vorliegende Erfindung betrifft eine isolierte Nukleinsäure zum Inhibieren des Schossens und Blühens einer Zuckerrübenpflanze sowie deren Verwendung, ein Protein, ein Verfahren zur Herstellung einer transgenen Zuckerrübenpflanze, bei der das Schossen und Blühen nach Vernalisation inhibiert ist, Vektoren oder mobile genetische Elemente sowie eine transgene Zuckerrübe, bei der das Schossen und Blühen nach Vernalisation inhibiert ist, und Samen und Teile davon.

Mittels molekularbiologischer Methoden ist es möglich, Nutzpflanzen genetisch zu verändern, ihre Eigenschaften damit zu ändern und zu verbessern. Eine für den Anbau und die Nutzung wichtige Eigenschaft von zweijährigen Pflanzen wie der Zuckerrübe (Beta vulgaris) besteht darin, dass deren Schossen und anschließendes Blühen einer Induktion durch eine längere Kälteperiode, wie sie in gemäßigten Breiten regelmäßig im Winter auftritt, bedarf. Dieser durch eine andauernde Periode mit niedrigen Temperaturen hervorgerufene Wechsel von der vegetativen in die generative Phase wird als Vernalisation bezeichnet.

Es gibt verschiedene Stoffwechselwege, von denen das Blühen gesteuert wird. Dazu gehören u.a. der Langtagstoffwechselweg, ein autonomer, ein giberillinsäure- und ein vernalisationsabhängiger Stoffwechselweg. Eine große Anzahl von Genen, die an der Regulation des Blühens beteiligt sind, konnten in den letzten Jahren identifiziert werden. Besonders die Kontrolle des Blühzeitpunktes wurde extensiv an der Modellpflanze Arabidopsis erforscht (Boss, P.K., Bastow, R.M., Mylne, J.S., und Dean, C. (2004) Multiple pathways in the decision to flower: Enabling, promoting, and resetting, Plant Cell 16 Suppl: 18-31; He, Y. und Amasino, R. M. (2005) Role of chromatin modification in flowering-time control, Trends Plant Sci 10, 30-35; Bäurle, I. und Dean, C. (2006) The timing of developmental transitions in plants, Cell 125(4): 655-664). Vor allem mittels Arabidopsis-Mutantenanalyse konnten viele "Frühblüh"("Early-flowering")- oder "Spätblüh"("Late-flowering")-Gene identifiziert werden (Gazzani S., Gendall, A.R., Lister, C., und Dean, C. (2003) Analysis of the molecular basis of flowering time variation in Arabidopsis accessions, Plant Physiol. 132: 1107-1114; Geraldo, N., Bäurle, I., Kidou, S., Hu, X., und Dean, C. (2009), FRIGIDA Delays Flowering in Arabidopsis via a Cotranscriptional Mechanism Involving Direct Interaction with the Nuclear Cap-Binding Complex, Plant Physiology, Juli 1, 2009; 150(3): 1611-1618; Michaels, SD, Amasino, RM (2001) Loss of FLOWERING LOCUS C activity eliminates the late-flowering phenotype of FRIGIDA and autonomous pathway mutations but not responsiveness to vernalization, Plant Cell 13: 935-942).

In Zuckerrübe wurde bisher nur BvFLC eingehend charakterisiert. Dabei hat sich gezeigt, dass dieses Gen nicht als Schlüsselkontrollgen für den Blühstoffwechsel bzw. die Vernalisation der Zuckerrübe dient (Reeves, PA, He, Y, Schmitz, RJ, Amasino, RM, Panella, LW, Richards, CM (2007), Evolutionary conservation of the FLOWERING LOCUS C-mediated vernalization response: evidence from the sugar beet (Beta vulgaris), Genetics 176(1): 295-307; Chia, T. Y. P., Müller, A., Jung, C., und Mutasa-Göttgens, E. S. (2008), Sugar beet contains a large CONSTANS-LIKE gene family including a CO homologue that is independent of the earlybolting (B) gene locus, J. Exp. Bot. 59(10): 2735-2748).

Das Schossen und Blühen von Zuckerrübenpflanzen ist unerwünscht, da in diesem Fall nicht die Samen bzw. Früchte, sondern der unterirdische Teil der Pflanze, die Rübe, genutzt wird, deren Speicherstoffe jedoch während des Schossens und Blühens von der Pflanze aufgebraucht würden. Darüber hinaus kann bei einzelnen Pflanzen, die als "Schosser" bezeichnet werden, ein unerwünschtes Schossen bereits im Jahr der Aussaat auftreten, was bei Ernte und Verarbeitung sehr nachteilig ist.

Aufgabe der vorliegenden Erfindung ist es daher, Mittel bereitzustellen, die es ermöglichen, das Schossen und/oder Blühen der Zuckerrübe zu inhibieren und sogar vollständig zu verhindern.

Erfindungsgemäß erfolgt die Lösung der gestellten Aufgabe mittels einer isolierten Nukleinsäure, wobei die Nukleinsäure eine Nukleotidsequenz umfasst, die
a) eine Sequenz der SEQ ID NO: 1-3 oder eine Teilsequenz der SEQ ID NO: 1-3, die mindestens 20 aufeinander folgende Nukleotide umfasst, aufweist, oder
b) zu einer Sequenz der SEQ ID NO: 1-3 oder zu einer Teilsequenz der SEQ ID NO: 1-3, die mindestens 20 aufeinander folgende Nukleotide umfasst, komplementär ist, oder
c) eine Sequenz der SEQ ID NO: 1-3 oder eine Teilsequenz der SEQ ID NO: 1-3, die mindestens 20 aufeinander folgende Nukleotide umfasst, oder eine dazu komplementäre Sequenz in Antisinn-Richtung aufweist, oder
d) zu einer Sequenz der SEQ ID NO: 1-3 zu mindestens 90% identisch ist oder zu einer Teilsequenz der SEQ ID NO: 1-3 mit einer Sequenzlänge von mindestens 100 Nukleotiden zu mindestens 90% identisch ist oder zu einer Teilsequenz der SEQ ID NO: 1-3 mit einer Sequenzlänge von 50-99 Nukleotiden zu mindestens 95% identisch ist, oder
e) für ein Protein mit der Aminosäuresequenz der SEQ ID NO: 4 oder einen Teil des Proteins, welches mindestens 30 aufeinander folgende Aminosäuren der SEQ ID NO: 4 umfasst, kodiert, oder
f) für ein Protein mit einer Aminosäuresequenz aus Beta vulgaris kodiert, die zu der Sequenz der SEQ ID NO: 4 zu mindestens 90% identisch oder zu einem Sequenzabschnitt, der mindestens 50 aufeinander folgende Aminosäuren der SEQ ID NO: 4 umfasst, zu mindestens 90% identisch ist, oder
g) mit einer Sequenz gemäß SEQ ID NO: 1-3 oder einer dazu komplementären Nukleotidsequenz oder einer dazu in Antisinn-Richtung orientierten Nukleotidsequenz unter stringenten Bedingungen hybridisiert und die hybridisierenden Sequenzen mindestens 90% Sequenzidentität aufweisen, wobei die stringenten Bedingungen Hybridisieren in 4 x SSC bei 65 °C und anschließendes mehrfaches Waschen in 0,1 x SSC bei 65 °C für insgesamt etwa 1 Stunde sind.

Die erfindungsgemäße Nukleinsäure kann dazu verwendet werden, beispielsweise mittels RNAi-Ansatz oder miRNA-Ansatz (Fire, A, Xu, S, Montgomery, M, Kostas, S, Driver, S, Mello, C (1998). Potent and specific genetic interference by double-stranded RNA in Caenorhabditis elegans, Nature 391 (6669): 806-811) das Schossen und Blühen von Zuckerrüben zu hemmen, vornehmlich, das Schossen und Blühen nach Möglichkeit vollständig zu unterbinden, z.B. durch Inhibieren von Genen, die Blühinduktoren wie FT, FUL, Co oder VIN3 kodieren.

Die erfindungsgemäße Nukleinsäure zeichnet sich besonders dadurch aus, dass sich mit ihr transgene Pflanzen, insbesondere Zuckerrüben, mit besonderen Eigenschaften herstellen lassen: In bevorzugter Weise lässt sie sich beispielsweise zu folgenden Zwecken bzw. mit folgenden Vorteilen verwenden:
Herstellung nichtschossender, nichtblühender Zuckerrüben
Herstellung einer Zuckerrübe als Winterrübe
Herstellung einer Zuckerrübe als Lenzrübe
Erhöhung der Biomasse von Zuckerrüben
Erhöhung des Zuckerertrages
Vermeidung der Schosserbildung bei Zuckerrüben
Verlängerung der Zuckerrübenkampagne
Vermeidung von Speichersubstanzverlusten bei Zuckerrüben
Nutzung der höheren Feuchte im Herbst
Bedeckung des Bodens und Nutzung des eingelagerten Stickstoffs
Schutz für Nützlinge auf dem Feld

Die Zuckerrübe ist eine zweijährige Pflanze. Nach Überwinterung und damit erfolgter Vernalisation kommt die Zuckerrübe normalerweise im zweiten Jahr zur Blüte. Mittels der erfindungsgemäßen Nukleinsäure, zum Beispiel einer in einem RNAi- oder MicroRNA-Ansatz eingesetzten Sequenz gemäß SEQ ID No: 5 bis SEQ ID No: 7 oder einer anderen erfindungsgemäßen Sequenz oder Teilsequenz, können Gene inhibiert und die Effekte der Vernalisation inhibiert bzw. gänzlich unterbunden werden. Mechanismen und Methoden zur Inhibierung bzw. Abschaltung von Genen sind dem Fachmann beispielsweise unter dem Begriff "Gene silencing" bekannt und können die bereits erwähnten und dem Fachmann geläufigen RNAi- oder Micro(mi)RNA-Verfahren umfassen, sind aber hierauf nicht beschränkt. In einem RNAi-Ansatz können beispielsweise die Sequenzen gemäß SEQ ID No: 5 bis SEQ ID No: 7 mittels dem Fachmann bekannter molekularbiologischer Techniken in Antisinnorientierung unter Kontrolle eines geeigneten Promotors in eine Zuckerrübenzelle eingebracht und dort exprimiert werden.

Durch die vorliegende Erfindung kann das Schossen und Blühen der Pflanze vollständig unterbunden werden. Das Rübensaatgut kann dadurch früher ausgebrächt werden, was letztlich zu einer verlängerten Vegetationsperiode und damit zu einer höheren Biomasse bzw. einem höheren Zuckerertrag führt. In Kombination mit einer Kältetoleranz können die Rüben beispielsweise auch als so genannte Winterrüben angebaut werden. Bei einem Anbau der Zuckerrübe im August kann die Rübe im darauf folgenden Frühjahr bereits als Lenzrübe geerntet werden. Dies ermöglicht dem Landwirt eine zusätzliche Fruchtfolge. Durch Verwendung der erfmdungsgemäßen Nukleinsäure führen längere Kälteeinbrüche nach Aussaat auf dem Feld nicht mehr zu verstärkter Schosserbildung. Auch die ohne längere Kälteeinbrüche bei bisherigen Zuckerrüben beobachtbare Schosserbildung kann verhindert oder zumindest deutlich vermindert werden. Mit Hilfe der vorliegenden Erfindung kann nicht nur bewirkt werden, dass das Schossen und in der Folge davon auch das Blühen von Zuckerrüben nach einer ersten Vernalisation, d.h. im zweiten Jahr, inhibiert bzw. verhindert wird, sondern die Zuckerrübe auch weiteren Kälteperioden ausgesetzt werden kann, ohne dass Vernalisationswirkungen beobachtbar wären.

Der Zuckerrübenanbau erfolgt normalerweise von April bis Oktober/November. Da nicht alle geernteten Zuckerrüben zur gleichen Zeit verarbeitet werden können, müssen diese ein- bzw. zwischengelagert werden. Während der Lagerung z.B. in Mieten entstehen große Speichersubstanzverluste (Saccharoseverluste) durch Spaltung der Saccharose in Glucose und Fruktose. Mittels der erfindungsgemäßen Nukleinsäure, insbesondere bei deren Verwendung in einem RNAi Ansatz, können die Aussaat und Erntetermine so variiert werden, dass die Gesamterntezeit (Kampagne) ohne Ernteverluste verlängert werden kann. Es können damit mehr Zuckerrüben während eines längeren Zeitraums ohne oder mit geringem Speichersubstanzverlust verarbeitet werden.

Unter dem Begriff "Zuckerrübe" oder "Zuckerrübenpflanze" wird hier eine Pflanze der Gattung Beta vulgaris verstanden, z.B. Beta vulgaris ssp. vulgaris var. altissima (Zuckerrübe i.e.S.), Beta vulgaris ssp. maritima (See-Mangold), Beta vurlgaris ssp. vulgaris var. vulgaris (Mangold), Beta vulgaris ssp. vulgaris var. conditiva (Rote Rübe/Bete), Beta vulgaris ssp. vulgaris var. crassa/alba (Futterrübe).

Unter einer "isolierten Nukleinsäure" wird eine aus ihrer natürlichen bzw. ursprünglichen Umgebung herausgelöste Nukleinsäure verstanden. Der Begriff umfasst auch eine synthetisch hergestellte Nukleinsäure.

Unter einer "Inhibierung des Schossens und Blühens" einer Zuckerrübenpflanze wird hier eine Minderung des Anteils von schossenden und gegebenenfalls blühenden Zuckerrübenpflanzen im Vergleich zu einer nicht erfindungsgemäß veränderten Zuckerrübenpflanze in einer vergleichbaren Entwicklungsphase, insbesondere im zweiten Jahr nach Durchlaufen einer entsprechenden Kälteperiode, d.h. nach Vernalisation, verstanden. Insbesondere umfasst der Begriff eine Minderung des Schosseranteils auf höchstens 80%, vorzugsweise höchstens 70%, 60%, 50%, 40%, 30%, 20% oder 10%, besonders bevorzugt höchstens 5%, 4%, 3%, 2%, 1%, 0,5%, 0,4%, 0,3%, 0,2% oder 0,1% des Schosseranteils von nicht gemäß der Erfindung veränderten Kontrollpflanzen. "Kontrollpflanzen" sind vorzugsweise Pflanzen derselben Sorte, die jedoch nicht gemäß der vorliegenden Erfindung verändert sind, und die beispielsweise einen Schosseranteil von maximal 0.01% aufweisen. Unter dem Begriff "Unterbindung" oder "vollständige Unterbindung" des Schossens und Blühens wird eine Inhibierung um mindestens 99%, vorzugsweise mindestens 99,5%, besonders bevorzugt mindestens 99,8% oder mindestens 99,9%, d.h. eine Minderung des Schosseranteils auf höchstens 1%, höchstens 0,5%, höchstens 0,2% oder höchstens 0,1%, insbesondere im zweiten Jahr nach Vernalisation, im Vergleich zu einer nicht erfindungsgemäß veränderten Zuckerrübenpflanze, die beispielsweise einen Schosseranteil von maximal 0,01 % aufweist, verstanden. Der Begriff der Inhibierung bzw. des Unterbindens des Schossens und Blühens umfasst vor allem die Inhibierung/Unterbindung des Schossens, unabhängig davon, ob es zu einem Blühen der Pflanze kommt, oder nicht.

Der Begriff "transgen" bedeutet hier gentechnisch verändert. Der Begriff umfasst, wenn er hier verwendet wird, auch den Fall, dass eine arteigene Nukleinsäure in einer Form, Anordnung oder Menge in die Zelle eingebracht ist, in der die Nukleinsäure natürlicherweise nicht in der Zelle vorkommt.

Der Begriff "Homologie" bezieht sich auf Übereinstimmungen oder Ähnlichkeiten in der Nukleotidsequenz zweier Nukleinsäuremoleküle oder Aminosäuresequenz zweier Proteine bzw. Peptide. Das Vorhandensein einer Homologie zwischen zwei Nukleinsäuren bzw. Proteinen kann festgestellt werden, indem man jeweils eine Position in der einen Sequenz mit der entsprechenden Position in der anderen Sequenz vergleicht und ermittelt, ob hier identische oder ähnliche Reste vorhanden sind. Zwei miteinander verglichene Sequenzen sind homolog, wenn ein bestimmter Mindestanteil identischer oder ähnlicher Nukleotide vorliegt. "Identisch" heißt, dass beim Vergleich zweier Sequenzen an äquivalenten Stellen jeweils dasselbe Nukleotid bzw. dieselbe Aminosäure steht. Dabei kann es gegebenenfalls erforderlich sein, Sequenzlücken zu berücksichtigen, um eine möglichst gute Alinierung der Vergleichssequenzen herzustellen. Ähnliche Nukleotide/Aminosäuren sind dabei nichtidentische Nukleotide/Aminosäuren mit gleichen oder äquivalenten chemisch-physikalischen Eigenschaften. Beim Austausch eines Nukleotids (einer Aminosäure) durch ein anderes Nukleotid (eine andere Aminosäure) mit gleichen oder äquivalenten chemisch-physikalischen Eigenschaften spricht man von einem "konservativen Austausch". Beispiele für physikalisch-chemische Eigenschaften einer Aminosäure sind beispielsweise die Hydrophobie oder die Ladung. In Zusammenhang mit Nukleinsäuren wird auch von einem ähnlichen Nukleotid bzw. einem konservativen Austausch gesprochen, wenn in einer kodierenden Sequenz ein Nukleotid in einem Kodon durch ein anderes ersetzt wird, wobei jedoch, z.B. aufgrund der Degeneration des genetischen Kodes, dieselbe Aminosäure oder eine ähnliche Aminosäure wie in der Vergleichssequenz von dem vom Austausch betroffenen Kodon kodiert wird. Dem Fachmann ist bekannt, welcher Nukleotid- bzw. Aminosäureaustausch ein konservativer Austausch ist. Zur Feststellung des Grades an Ähnlichkeit bzw. Identität zwischen zwei Nukleinsäuren wird dabei vorzugsweise von einer Mindestlänge von 60 Nukleotiden bzw. Basenpaaren, bevorzugt einer Mindestlänge von 70, 80, 90, 100, 110, 120, 140, 160, 180, 200, 250, 300, 350 oder 400 Nukleotiden bzw. Basenpaaren, besonders bevorzugt von der vollen Länge der zu vergleichenden Nukleinsäuren ausgegangen, bei Proteinen/Peptiden von einer Mindestlänge von 20 Aminosäuren, bevorzugt einer Mindestlänge von 25, 30, 35, 40, 45, 50, 60, 80, 100, 150, 200, 250 oder 300 Aminosäuren, und besondes bevorzugt von der vollen Länge der zu vergleichenden Aminosäuresequenzen ausgegangen. Der Grad der Ähnlichkeit ("positives") oder Identität zweier Sequenzen kann beispielsweise mit Hilfe des Computerprogramms BLAST (S.F. Altschul et al. (1990), Basic Local Alignment search tool, J.Mol. Biol. 215: 403-410; s. z.B. http://www.ncbi.nlm.nih.gov/BLAST/) unter Verwendung von Standardparametern ermittelt werden. Die Feststellung einer Homologie hängt von der Länge der verglichenen Sequenzen ab. Für die Zwecke der vorliegenden Erfindung wird von Homologie zwischen zwei Nukleinsäuresequenzen, deren kürzere mindestens 100 Nukleotide umfasst, ausgegangen, wenn mindestens 70%, mindestens 75%, mindestens 80%, mindestens 85%, mindestens 90%, mindestens 95%, mindestens 97%, mindestens 98%, oder mindestens 99% der Nukleotide identisch und/oder ähnlich ("identities" oder "positives" gemäß BLAST), vorzugsweise identisch, sind. Bei einer Sequenzlänge von 50-99 Nukleotiden wird bei einer Identität oder zumindest Ähnlichkeit von mindestens 80%, vorzugsweise mindestens 85%, 86%, 87%, 88% oder 89%, bei einer Sequenzlänge von 15-49 Nukleotiden bei einer Identität oder Ähnlichkeit von mindestens 90%, bevorzugt mindestens 95%, 96%, 97%, 98% oder 99% von Homologie zwischen den Sequenzen ausgegangen. Bei Proteinen wird von Homologie ausgegangen, wenn bei Verwendung des Computerprogramms BLAST unter Verwendung von Standardparametern und der BLOSUM62-Substitutionsmatrix (Henikoff, S., und Henikoff, J., Amino acid substitution matrices from protein blocks. Proc. Natl. Acad. Sci. USA. 89: 10915-10919, 1992) eine Identität ("identities") und/oder Ähnlichkeit ("positives"), vorzugsweise Identität, von mindestens 25%, mindestens 26%, mindestens 27%, mindestens 28%, mindestens 29%, mindestens 30%, vorzugsweise mindestens 35%, mindestens 40%, mindestens 45%, mindestens 50%, mindestens 55%, mindestens 60%, mindestens 65%, mindestens 70%, mindestens 75%, mindestens 80%, mindestens 85%, mindestens 90%, mindestens 95%, mindestens 97%, mindestens 98%, oder mindestens 99% erhalten wird, wobei vorzugsweise die gesamte Länge des Proteins/Peptids, das mit einem anderen Protein verglichen wird, berücksichtigt wird, z.B. die Länge von 653 Aminosäuren im Falle der SEQ ID NO: 4. Dem Fachmann ist anhand seines Fachwissens ohne weiteres ersichtlich, welches der verfiigbaren BLAST-Programme (z.B. BLASTn, BLASTp, BLASTx, tBLASTn oder tBLASTx) für die Ermittlung der Homologie in Frage kommt. Darüber hinaus existieren noch weitere Programme, die der Fachmann kennt, und die er im Bedarfsfall bei der Beurteilung der Homologie zweier oder mehrerer zu vergleichender Sequenzen oder Teilsequenzen heranziehen kann. Solche Programme sind beispielsweise auf den Internetseiten des European Bioinformatics Institute (EMBL) verfügbar (s. z.B. http://www.ebi.ac.uk/Tools/similarity.html).

Unter "Hybridisieren" oder "Hybridisierung" wird ein Vorgang verstanden, bei dem ein einzelsträngiges Nukleinsäuremolekül sich an einen komplementären Nukleinsäurestrang anlagert, d.h. mit diesem eine Basenpaarung eingeht. Standardverfahren zur Hybridisierung sind beispielsweise in Sambrook et al. (Molecular Cloning. A laboratory manual, Cold Spring Harbor Laboratory Press, 3. Aufl. 2001) beschrieben. Vorzugsweise wird darunter verstanden, dass mindestens 50%, weiter bevorzugt mindestens 55%, 60%, 65%, 70%, 75%, 80% oder 85%, besonders bevorzugt 90%, 91%, 92%, 93%, 94%, 95%, 96%, 97%, 98% oder 99% der Basen des Nukleinsäurestranges eine Basenpaarung mit dem komplementären Nukleinsäurestrang eingehen. Die Möglichkeit einer solchen Anlagerung hängt von der Stringenz der Hybridisierungsbedingungen ab. Der Begriff "Stringenz" bezieht sich auf die Hybridisierungsbedingungen. Hohe Stringenz ist dann gegeben, wenn eine Basenpaarung erschwert ist, niedrige Stringenz, wenn eine Basenpaarung erleichtert ist. Die Stringenz der Hybridisierungsbedingungen hängt beispielsweise von der Salzkonzentration bzw. Ionenstärke und der Temperatur ab. Generell kann die Stringenz durch eine Erhöhung der Temperatur und/oder eine Erniedrigung des Salzgehaltes erhöht werden. Unter "stringenten Hybridisierungsbedingungen" sind solche Bedingungen zu verstehen, bei denen eine Hybridisierung überwiegend nur zwischen homologen Nukleinsäuremolekülen stattfindet. Der Begriff "Hybridisierungsbedingungen" bezieht sich dabei nicht nur auf die bei der eigentlichen Anlagerung der Nukleinsäuren herrschenden Bedingungen, sondern auch auf bei den anschließenden Waschschritten herrschenden Bedingungen. Stringente Hybridisierungsbedingungen sind beispielsweise Bedingungen, unter denen überwiegend nur solche Nukleinsäuremoleküle hybridisieren, die mindestens 70%, vorzugsweise mindestens 75%, mindestens 80%, mindestens 85%, mindestens 90% oder mindestens 95% Sequenzidentität aufweisen. Wenig stringente Hybridisierungsbedingungen sind beispielsweise: Hybridisieren in 4 x SSC bei 37 °C und anschließendes mehrfaches Waschen in 1 x SSC bei Raumtemperatur. Stringente Hybridisierungsbedingungen sind beispielsweise: Hybridisieren in 4 x SSC bei 65 °C und anschließendes mehrfaches Waschen in 0,1 x SSC bei 65 °C für insgesamt etwa 1 Stunde.

Der Begriff "komplementär" bezieht sich auf die Fähigkeit von Purin- und Pyrimidinnukleotiden, über Wasserstoffbrückenbindungen miteinander Basenpaare zu bilden. Komplementäre Basenpaare sind beispielsweise Guanin und Cytosin, Adenin und Thymin sowie Adenin und Uracil. Ein komplementärer Nukleinsäurestrang ist dementsprechend ein Nukleinsäurestrang, der durch Paarung mit komplementären Basen eines anderen Nukleinsäurestrangs einen Doppelstrang bilden kann.

Unter einem "Fragment" oder einer "Teilsequenz" einer Nukleinsäure ist hier ein zusammenhängender Teilabschnitt der Nukleinsäure zu verstehen, d.h. ein Sequenzabschnitt aus aufeinander folgenden Nukleotiden der Nukleinsäure. Fragmente können z.B. in einem RNAi- oder miRNA-Ansatz vorteilhaft eingesetzt werden, wobei die Sequenz dabei beispielsweise in Antisinn("antisense")-Richtung eingesetzt werden kann. "Antisinnrichtung" bzw. "Antisinnorientierung" einer Nukleinsäuresequenz, z.B. einer DNA-Sequenz, bedeutet hier beispielsweise, dass eine Transkription der DNA-Sequenz zu einer mRNA führt, deren Nukleotidsequenz komplementär ist zu einer natürlichen (endogenen) mRNA, so dass deren Translation durch Anlagerung der komplementären RNA behindert oder verhindert wird. Unter einer "Antisinn-RNA" bzw. "Antisense-RNA" wird eine zu einer bestimmten mRNA oder zu bestimmten anderen RNAs komplementäre RNA verstanden. "Antisinnrichtung" oder "Antisinnorientierung" einer mRNA-Sequenz bedeutet daher, dass die mRNA eine Sequenz aufweist, die komplementär ist zu einer mRNA-Sequenz, deren Translation somit durch Anlagerung behindert oder verhindert werden kann. Teilsequenzen, die im Rahmen der vorliegenden Erfindung vorteilhaft beispielsweise in Antisinnorientierung eingesetzt werden können, sind beispielsweise Nukleinsäuren mit einer Sequenz gemäß SEQ ID NO: 5, 6 oder 7. Bei diesen Teilsequenzen handelt es sich um Abschnitte der erfindungsgemäßen Nukleinsäure gemäß SEQ ID NO: 3. Es können aber auch beliebige andere Nukleinsäuren mit Sequenzen oder Teilsequenzen der SEQ ID 1-3, die mindestens 20 aufeinander folgende Nukleotide umfasst, zum Beispiel in Antisinnrichtung, eingesetzt werden.

Bevorzugt umfasst eine Teilsequenz einer Nukleinsäure mindestens 20, 25, 30, 35, 40, 45, 50, 60, 70, 80, 90 oder mindestens 100 aufeinander folgende Nukleotide, weiter bevorzugt mindestens 150, 200, 250, 300, 350, 400 oder 450 aufeinander folgende Nukleotide. Ein Teil eines Proteins (s. z.B. Buchstabe f) oben) umfasst vorzugsweise mindestens 30, 40 oder 50, besonders bevorzugt mindestens 60, 70, 80, 90 oder mindestens 100 aufeinander folgende Aminosäuren der SEQ ID NO: 4. Der Sequenzabschnitt der SEQ ID NO: 4 (s. z.B. Buchstabe g) oben) umfasst bevorzugt mindestens 50, 60, 70, 80 oder 90, weiter bevorzugt mindestens 100, 120, 150, 200 oder 250 aufeinander folgende Aminosäuren der SEQ ID NO: 4. Die erforderliche oder sinnvolle Länge der Teilsequenz der Nukleinsäure oder des Proteins oder des Sequenzabschnitts kann der Fachmann mit Hilfe seines allgemeinen Fachkönnens und gegebenenfalls unter Durchführung von Routineversuchen anhand des gewählten Ansatzes und der beabsichtigten Wirkung feststellen, ohne dass es hierzu einer erfinderischen Tätigkeit bedürfte.

Die erfindungsgemäße Nukleinsäure ist vorzugsweise zu mindestens 90%, 95%, 96%, 97%, 98% oder 99%, besonders bevorzugt mindestens 99,5%, 99,6%, 99,7%, 99,8% oder 99,9% zu einer Sequenz oder Teilsequenz der SEQ ID NO: 1-3 identisch.

In einem weiteren Aspekt betrifft die vorliegende Erfindung die Verwendung einer oder mehrerer der erfindungsgemäßen Nukleinsäuren zur Inhibierung des Schossens und Blühens einer Zuckerrübenpflanze. Wie bereits oben angegeben, kann eine erfindungsgemäße Nukleinsäure beispielsweise in Antisinnorientierung in eine Zuckerrübenpflanze eingebracht werden, um dadurch für eine Inhibierung der für die Induktion des Schossens und Blühens verantwortlichen Gene zu sorgen. Verfahren, die zur Einführung einer erfindungsgemäßen Nukleinsäure in eine Zuckerrübenzelle geeignet sind, sind dem Fachmann grundsätzlich bekannt, und umfassen beispielsweise die Agrobakterien-vermittelte Transformation. Das Einbringen einer Nukleinsäure in Antisinnorientierung in eine Pflanze ist allerdings nur eine der dem Fachmann bekannten Verfahren zur Hemmung oder Unterdrückung einer Genaktivität. Die erfindungsgemäßen Nukleinsäuren sind auch im Rahmen anderer Verfahren, oder Mechanismen, die eine Hemmung bzw. Unterdrückung des SchossensBlühens bewirken können, vorteilhaft einsetzbar.

Darüber hinaus können die erfindungsgemäßen Nukleinsäuren auch als Sonde zur Ermittlung weiterer Faktoren, Gene bzw. Genprodukte verwendet werden, mit deren Hilfe das Blühen und Schossen von Zuckerrübenpflanzen gehemmt und/oder unterdrückt werden kann.

In einem weiteren Aspekt betrifft die vorliegende Erfindung ein Protein mit einer Aminosäuresequenz der SEQ ID NO: 4 oder ein Protein mit einer Aminosäuresequenz, die einen Sequenzabschnitt der SEQ ID NO: 4, vorzugsweise mindestens 50, 60, 70, 80 oder 90, bevorzugt mindestens 100, 120, 150, 200 oder mindestens 250 aufeinander folgende Aminosäuren der SEQ ID NO: 4, umfasst, oder ein zu dem Protein mit der Aminosäuresequenz oder einem Sequenzabschnitt der SEQ ID NO: 4 homologes Protein aus Beta vularis. Das Protein oder ein beliebiger Teil davon, bzw. die entsprechenden Aminosäuresequenzen, kann/können beispielsweise als Sonde auf Aminosäureebene zur Ermittlung weiterer Faktoren, Gene bzw. Genprodukte verwendet werden, mit deren Hilfe das Blühen und Schossen von Zuckerrübenpflanzen gehemmt und/oder unterdrückt werden kann.

In noch einem weiteren Aspekt betrifft die vorliegende Erfindung auch ein Verfahren zur Herstellung einer transgenen Zuckerrübenpflanze, umfassend die Schritte (a) des Transformierens einer Zuckerrübenzelle mit einer oder mehreren erfindungsgemäßen Nukleinsäuren und (b) des Heranziehens einer Zuckerrübenpflanze aus der transformierten Zuckerrübenzelle. Die Transformation der Zuckerrübenzelle kann beispielsweise mit Hilfe bekannter Vektoren, z.B. eines Ti-Plasmids, erfolgen, und ist dem Fachmann grundsätzlich bekannt. Die erfindungsgemäßen Nukleinsäuren können in einem solchen Vektor vorteilhaft unter die Kontrolle eines geeigneten Promotors gestellt sein.

Die Erfindung betrifft auch einen Vektor oder ein mobiles genetisches Element, das eine oder mehrere erfindungsgemäße Nukleinsäuren umfasst. Vektoren und mobile genetische Elemente sind dem Fachmann bekannt und umfassen zum Beispiel Plasmide wie das Ti-Plasmid. Der Vektor bzw. das mobile genetische Element kann vorteilhaft Kontrollelemente, z.B. einen Promotor, enthalten.

Darüber hinaus betrifft die Erfindung auch eine Zuckerrübenpflanze, die eine oder mehrere erfindungsgemäße Nukleinsäuren, vorzugsweise unter der Kontrolle eines geeigneten Promotors, umfasst, und bei der das Schossen und Blühen inhibiert ist, sowie Samen und/oder Teile einer erfindungsgemäßen Zuckerrübenpflanze, die mit einer oder mehreren erfindungsgemäßen Nukleinsäuren transformiert sind und die eine oder mehreren Nukleinsäuren als Transgen enthalten.

Die Erfindung wird im Folgenden anhand von Ausführungsbeispielen und der beigefügten Figuren rein zu Veranschaulichungszwecken näher erläutert.

### Kurze Beschreibung der Figuren

Fig. 1 Schematischer Vergleich (BLAST) der jeweiligen Lage der PHD-, FNIII- und VID-Domänen in BvVIL, AtVIL1 und AtVIN3. Angegeben ist auch der Anteil identischer bzw. ähnlicher Nukleotide ("identities"/"positives", in %). VIN3_Ath = AtVIN3, VIL1_Ath = At-VIL1, VIL_Bv = BvVIL
Fig. 2 Schoss- und blühresistente Zuckerrübe. Links: Schossresistente BvVIL-Transformante WB4-7 (im Bild links) im Vergleich mit schossender Kontrolle (im Bild rechts); Rechts: BvVIL-Transformante WB4-7, mehrere Monate alt
Fig. 3 Schematische Darstellung zum Aufbau der verwendeten RNAi-Kassette.
Fig. 4-6 Schematische Darstellung der zur Erstellung des pRNAi-Vektors verwendeten Fragmente Sal-SMA-775>1077 (Fig. 4), Sal-SMA-779>1197 (Fig. 5) und Sal-SMA-971>1466 (Fig. 6).
Fig. 7-9 Schematische Darstellung der zur Herstellung von RNAi-Konstrukten verwendeten Vektoren pRNAi-bvvil-775>1077sas (Fig. 7), pRNAi-bvvil-779>1197sas (Fig. 8) und pRNAi-bvvil-971>1466sas (Fig. 9).

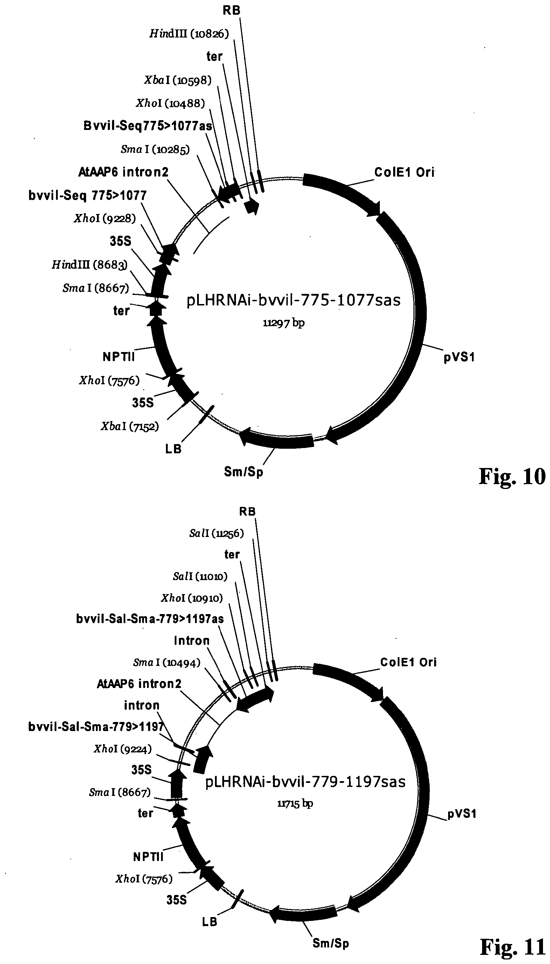

Fig. 10-12 Schematische Darstellung der für die Agrobakterien-vermittelte Transformation eingesetzten binären Ti-Plasmide pLHRNAi-bvvil-775-1077sas (Fig. 10), pLHRNAi-bwil-779-1197sas (Fig. 11) und pLHRNAi-bvvil_971-1446sas (Fig. 12).
Fig. 13 Schematische Darstellung der Intron-Exon-Struktur der genomischen DNA von BvVIL, mit Angabe von Restriktionsschnittstellen.
Fig. 14 Struktur der BvVIL-cDNA-Sequenz

### Ausführungsbeispiele

### Identifizierung/Isolierung einer vollständigen cDNA der Zuckerrübe für das Unterbinden des Schossens und Blühens

Durch Analysen einer eigens hierfür erstellten proprietären Zuckerrüben-EST-Datenbank wurde eine 1499bp lange, unvollständige cDNA (SEQ ID NO:3) identifiziert. Ausgehend von diesem unvollständigen cDNA-Bank-Klon wurde diese Sequenz vervollständigt. Die erhaltene Sequenz der c-DNA (SEQ ID NO:2) umfasste 1962bp (2285bp einschließlich 3'UTR). Das daraus resultierende Protein umfasst 653 Aminosäuren (SEQ ID NO:4). Die zugehörige genomische DNA-Sequenz (SEQ ID NO:1) umfasst 2366bp.

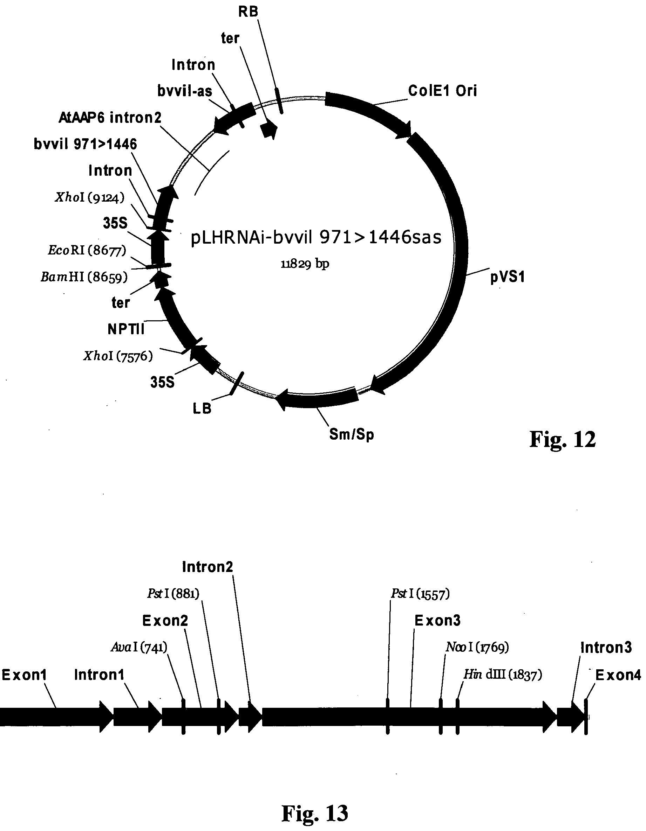

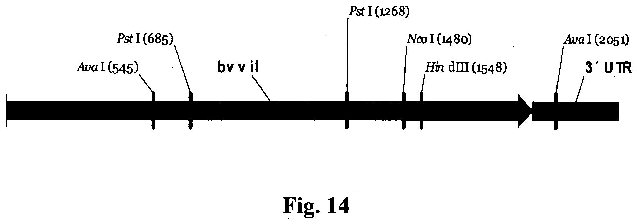

Ein Alignment der genomischen DNA mit cDNA zeigt den Aufbau der gesamten DNA, die aus 4 Exons und 3 Introns besteht (Fig. 13). Eine schematische Darstellung der cDNA ist in Fig. 14 zu finden.

### Charakterisierung/Annotierung der Sequenz

Ein Vergleich (BLAST) des erhaltenen Volllängen-Klons bzw. der übersetzten Protein-Sequenz zeigt eine nur geringe Sequenzähnlichkeit mit Arabidopsis-"Blüh-Genen" (z.B. At-VIL- und AtVIN-Familie). Dazu gehören die Gene VIN3 (VRN7), VIL1 (VRN5), VIL2 (VEL1), VIL3 (VEL2) und VIL4 (VEL3). Die höchste Ähnlichkeit über die gesamte Sequenzlänge besteht zu AtVIL1 mit 57,2% (s. Tabelle 1).

**Tabelle 1: Sequenzvergleich von Bv-VIL mit Arabidopsis-thaliana(At)-VIL-Kandidaten auf DNA-Ebene. Der Vergleich erfolgte bei At-VIN3 über eine Länge von 2343 bp, bei At-VIL1 über eine Länge von 2163 bp, bei At-VIL2 über eine Länge von 2555 bp, bei At-VIL3 über eine Länge von 1700 bp, und bei At-VIL4 über eine Länge von 1251 bp mit Hilfe des Programms AlignX (ClustalW).**

| | AtVIN3 | AtVIL1 | AtVIL2 | AtVIL3 | AtVIL4 |
|---|---|---|---|---|---|
| BvVIL | 46,0% | 57,2% | 51,5% | 49,8% | 45,9% |

Auch der Vergleich der Sequenzen auf Proteinebene mit der AtVIL- und -VIN-Genfamilie sowohl über die gesamte Sequenz als auch fokussiert auf die verschiedenen Domänen (charakteristisch für die VIL- und VIN-Genfamilie sind die PHD-, die FNIII- und die VID-Domänen) und die Bereiche zwischen den Domänen zeigt ebenfalls eine geringe Homologie (s. Tabelle. 2).

**Tabelle 2: BvVIL-Sequenzvergleich auf Proteinebene mit VIL-Genen aus Arabidopsis (Vergleich mit der Gesamt-Sequenzen und einzelnen Domänen). At = Arabidopsis thaliana, Bv = Beta vulgaris, Anm. = Anmerkung, AS = Aminosäuren, ident. = identisch ("identities"), pos. = ähnlich ("positives"), Kons.-L. AS = Konsensus-Länge AS, gaps = Lücken**

| | **Gesamt** | | | **Domänenverpleiche** | | | | | | | | | | | | |
|---|---|---|---|---|---|---|---|---|---|---|---|---|---|---|---|---|
| | | | | **Prä-PHD** | | **PHD-Domäne** | | **Inter-PHD-FN3** | | **FN3** | | **Inter-FN3-VID** | | | **VID** | |
| | **ident. %** | **pos. %** | **Kons.-L. AS** | **ident. %** | **pos. %** | **ident. %** | **pos. %** | **ident. %** | **pos. %** | **ident. %** | **pos. %** | **ident. %** | **pos %** | **Anm** | **ident. %** | **pos. %** |
| AtVIL1 | 46,1 | 57,3 | 641 | 28,8 | 48,5 | 66,7 | 78,8 | 58,9 | 67,9 | 44,7 | 62,4 | 34,9 | 41,3 | | 49,6 | 61,7 |
| AtVIL2 | 30,9 | 42,8 | 688 | 34,7 | 43,2 | 50,8 | 62,3 | 39,4 | 53,8 | 24,7 | 43,5 | 13 | 21,9 | | 41,6 | 58,4 |
| AtVIL3 | 23,4 | 33,6 | 692 | 16,4 | 25,9 | 55,2 | 65,7 | 36,3 | 46 | 20,7 | 42,5 | 3,6 | 8,3 | gaps | 44 | 55 |
| AtVIN3 | 28,6 | 39,2 | 696 | 27,6 | 30,1 | 50 | 62,1 | 46,4 | 67,9 | 21,2 | 40 | 11,9 | 18 | gaps | 40,2 | 54,9 |

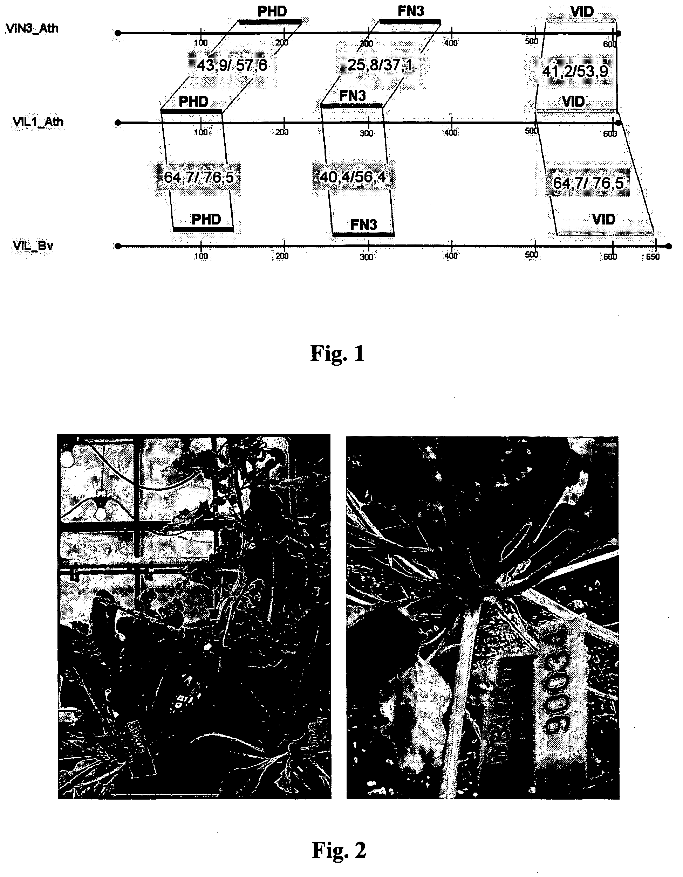

Ein Vergleich (BLAST) der einzelnen Domänen zeigt, dass es sich bei der neuen Sequenz um eine VIL-ähnliche Sequenz handelt. (Fig. 1). Die Sequenz (SEQ ID NO: 1) wird hier deshalb als BvVIL (Beta vulgaris vernalisation vin3-like) bezeichnet.

Ein Sequenzvergleich BvVIL mit AtVIL-Kandidaten auf Gesamt-DNA-Ebene zeigt jedoch nur eine maximale Homologie von 57,2 % mit AtVIL1. Mit anderen At-Genen der VIL-Familie zeigt sich eine noch geringere Sequenzähnlichkeit.

Eine Analyse nichttransgener Zuckerrüben auf Transkription von BvVIL vor, während und nach Vernalisation ergab, dass das Gen nicht nur nach Vernalisation transkribiert wird, sondern bereits vor, während und nach der Vernalisation konstitutiv aktiv ist. Im Gegensatz dazu wird beispielsweise VIN3 erst durch eine lang anhaltende Vernalisation aktiviert.

### Herstellung von RNAi-Konstrukten

Für die Herstellung von RNAi Konstrukten wurden drei Bereiche innerhalb der SEQ ID NO:3 verwendet:
Nukleotide 775 bis 1077 (SEQ ID NO: 5)
Nukleotide 779 bis 1197 (SEQ ID NO: 6)
Nukleotide 971 bis 1446 (SEQ ID NO: 7)

Die Sequenz gemäß SEQ ID NO: 5 wurde mittels des Primerpaares F_PLT3∼1:775U27, CTgggATACTTgggTgTTggAAAAAgC (SEQ ID NO: 8) und F_PLT3∼1:1050L28, TAgA-AACATTggCgAgCCATTCATTAgC (SEQ ID NO: 9) über PCR amplifiziert.

Die Sequenz gemäß SEQ ID NO: 6 wurde mittels des Primerpaares F_PLT3∼1:779U24, gA-TACTTgggTgTTggAAAAAgCA (SEQ ID NO: 10), und F_PLT3∼1:1175L23 AATCAgT-CATTggTgTggggATA (SEQ ID NO: 11) über PCR amplifiziert.

Die Sequenz gemäß SEQ ID NO 7 wurde mittels des Primerpaares F_PLT3∼1:971U21, CAAgATggCCAgAggTATTgT (SEQ ID NO: 12) und F_PLT3∼1:1426L21, CTTCCTTTTTA-CAgCCCACTg (SEQ ID NO: 13) über PCR amplifiziert.

Die PCR wurde unter Verwendung von 10 ng genomischer Zuckerrüben-DNA, einer Primerkonzentration von 0,2 µM bei einer "Annealing"-Temperatiur von 55 °C in einem Multicycler PTC-200 (MJ Research, Watertown, USA) durchgeführt. Die PCR-Produkte wurden jeweils in den Vektor pRTRNAi (Hirner A, Ladwig F, Stransky H, Okumoto S, Keinath M, Harms A, Frommer WB, Koch W. (2006) Arabidopsis LHT1 Is a High-Affinity Transporter for Cellular Amino Acid Uptake in Both Root Epidermis and Leaf Mesophyll Plant Cell. 18(8): 1931-46) in einer Inverted-Repeat-Struktur integriert. Der Vektor basiert auf dem Plasmid pRT100 und ist konstruiert für die Herstellung von "intronspliced" Hairpinstrukturen. Der Vektor enthält den 35S-Promoter für eine konstitutive Expression (Odell, J. T. Nagy, F., und Chua, N.-H. (1985) Identification of DNA sequences required for activity of the cauliflower mosaic virus 35S promoter, Nature 313, 810-812), das ATAAP6-Intron aus Arabidopsis und einen PolyA-Terminator. Das ATAAP6-Intron ist flankiert von den Schnittstellen Xho1/Ec1/136II am 5'-Ende bzw. von den Restriktionsschnittstellen Smal/Sall am 3'-Ende. Dies ermöglicht die Integration von identischen Fragmenten "in sense" und "antisense", wenn diese Fragmente die kompatiblen Restriktionsschnittstellen Xhol oder Sal1 besitzen, bzw. am anderen Ende stump ("blunt end") sind. Hierfür wurden die ursprünglichen PCR-Produkte mit neuen um diese Restriktionsschnittstellen verlängerten PCR-Primern reamplifiziert: Sal-SMA-775>1077 gagaggacgtcgacctgggatacttgggtg (SEQ ID NO: 14) und ccccccgggtagaaacattggcgagc (SEQ ID NO: 15), SAL-SMA-779>1197 gagaggacgtcgacgatacttgggtgttgg (SEQ ID NO: 16) und ccccccgggaatcagtcattggtgtggggata (SEQ ID NO: 17), XHO-SMA-971>1446 gagaggacctcgagcaagatggccagaggt (SEQ ID NO: 18) und gtcgacccccccgggcttccttttt (SEQ ID NO: 19). Zur weiteren Verwendung wurden die PCR Fragmente in den TA-Klonierungsvektor pCR2.1 (TOPO TA Cloning Kit (Invitrogen, Carlsbad, USA)) kloniert und in E. coli transformiert. Eine Blau-Weiss Selektion ermöglichte die Identifizierung rekombinanter Plasmide (Sambrook, J., Fritsch, E.. F., und Maniatis, T, 1989, In Molecular Cloning, A Laboratrory Manual, Cold Spring Harbor Laboratory Press, New York). Bei den weißen Kolonien ist durch ein Insert die Expression der ss-Galaktosidase unterdrückt, was zu weißen Kolonien führt, weil das dem Medium zugefügt Enzymsubstrat nicht mehr gespalten wird. Eine anschließende Sequenzierung mit M13-fwd/rev-Primern wurde bei Eurofins MWG Operon (Ebersberg, Deutschland) durchgeführt. Die Analyse und das Alignment der Sequenzdaten erfolgte über das Programm Vektor NTI (Invitrogen, Carlsbad, USA).

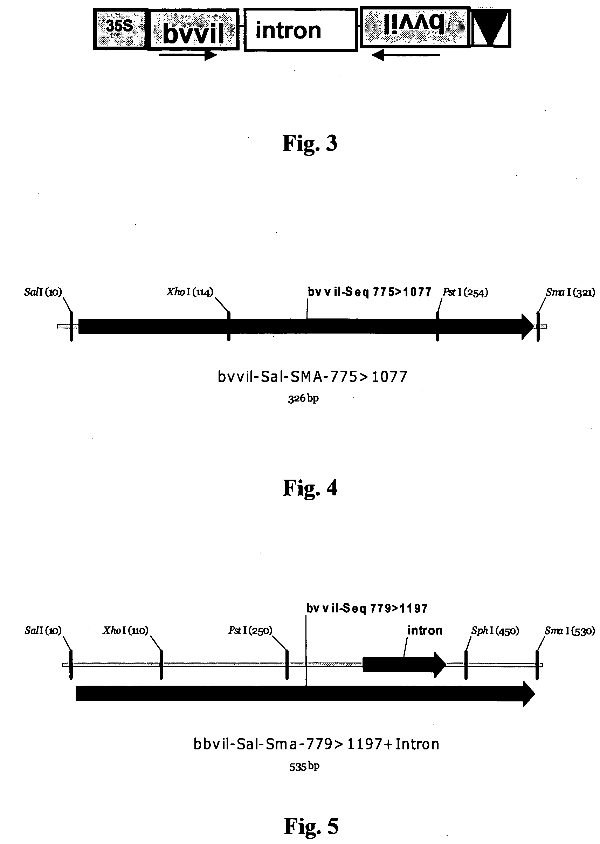

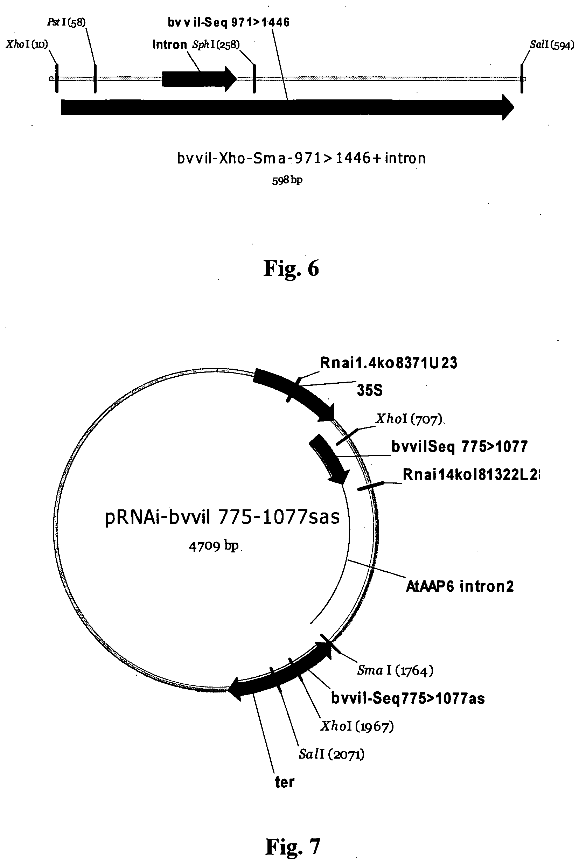

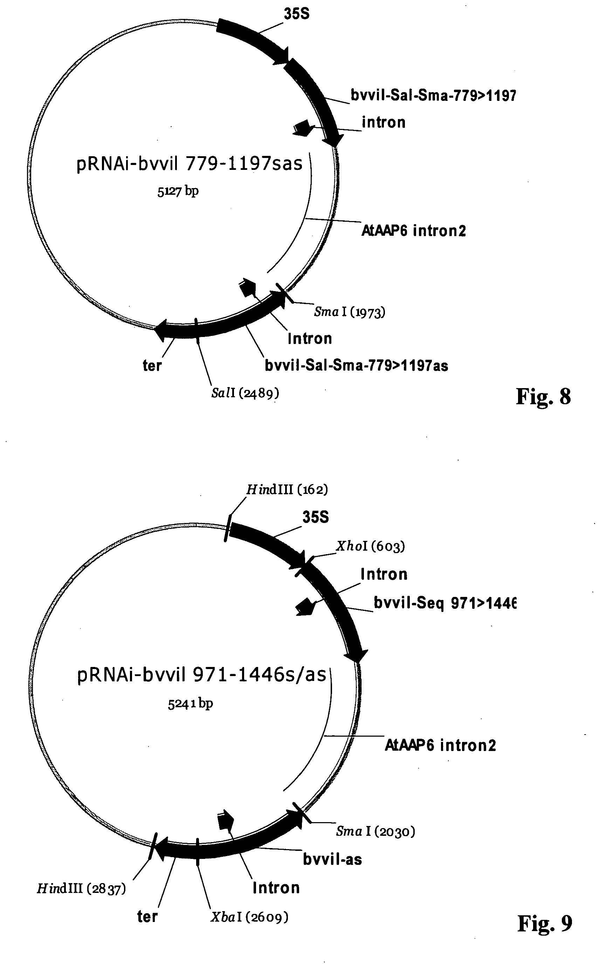

Die Fragmente Sal-SMA-775>1077, SAL-SMA-779>1197 und XHO-SMA-971>1446 (s. Fig. 4-6) wurden jeweils mittels Sall/Smal bzw. Xhol/Smal aus dem Topovektor herausgeschnitten und anschließend zuerst in den mit Sall/Smal bzw. Xhol/Ecl 136II aufgeschnittenen pRTRNAi Vektor "in sense" ligiert. Anschließend wurden die gleichen Fragmente ein zweites Mal in "antisense" in die kompatiblen Xhol/Ecl 136II bzw. Sall/Smal religiert. Die Klonierungen resultierten in den Vektoren pRNAi-bwil-775-1077sas, pRNAi-bwil-779-1197sas und pRNAi-bwil_971-1446sas (Fig. 3, 7-9).

### Herstellung von Transformationskonstrukten

Für die Herstellung von Transformationskonstrukten wurde der binäre Transformationsvektor pLH9000 (Hausmann, L. und Töpfer, R. (1999) Entwicklung von Plasmid-Vektoren. Vorträge zur Pflanzenzüchtung: Bioengineering für Rapssorten nach Maß 45: 155-172) (NCBI-Zugriffsnr.: AF458478) verwendet. Die Expressionskassetten wurden nach Restriktion der pRNAi-Plasmide mit HindIII isoliert, in den binären Vektor pLH9000 kloniert, und die resultierenden Plasmide wurden mit pLHRNAi-bvvil-775-1077sas, pLHRNAi-bvvi1-779-1197sas, und pLHRNAi-bvvil-971-1446sas bezeichnet.

### Zuckerrüben-Transformation und Überprüfung der Transgenität

Für die Pflanzentransformation fanden die binären Vektoren pLHRNAi-bvvi1-775-1077sas, pLHRNAi-bvvil-779-1197sas und pLHRNAi-bvvil-971-1446sas Verwendung. Die binären Vektoren wurden in den Agrobacterium-tumefaciens-Stamm C58C1 mit dem residenten Plasmid pGV3101 durch ein direktes DNA-Transformationsverfahren (An, G. (1987), Binary Ti vectors for plant transformation and promoter analysis, Methods Enzymol. 153, 292-305) transformiert. Die Selektion rekombinanter A.-tumefaciens-Klone erfolgte unter Verwendung des Antibiotikums Streptomycin (50 mg/l). Die Transformation der Zuckerrüben erfolgte nach Lindsey et al. (1991) unter Verwendung des Antibiotikums Kanamycin (Lindsey, K., Gallois, P., Eady, C. (1991), Regeneration and transformation of sugar beet by Agrobacterium tumefaciens, Plant Tissue Culture Manual B7: 1-13; Kluwer Academic Publishers). Die Transgenität der Pflanzen wurde durch PCR überprüft. Die Verwendung der Primer GTGGA-GAGGCTATTCGGTA (SEQ ID NO: 20) und CCACCATGATATTCGGCAAG (SEQ ID NO: 21) führte zu der Amplifikation eines 553 bp großen DNA-Fragments aus dem nptll-Gen. Die PCR wurde unter Verwendung von 10 ng genomischer DNA, einer Primerkonzentration von 0,2 µM bei einer Annealingtemperatur von 55 °C in einem Multicycler PTC-200 (MJ Research, Watertown, USA) durchgeführt.

### Überprüfung des Blüh- und Schossverhaltens der Transformanten

Ca. 40 unabhängige Transformanten nebst nichttransgener isogener Kontrollen wurden in vitro vermehrt und ins Gewächshaus überführt. Nach einer Anpassungsphase wurden die Transformanten für 3 Monate bei 8°C in der Kühlkammer einer Vernalisation unterzogen (Simulation Winter). Anschließend wurden die Transformanten zusammen mit identisch behandelten nichttransgenen Kontrollpflanzen wieder zurück ins Gewächshaus (25 °C) überführt. Kurz nach Überführung, nach 10 Tagen bereits, begannen die Kontrollpflanzen zu schossen. Nach ca. 4 Wochen begannen die Kontrollpflanzen zu blühen. Im Gegensatz dazu zeigten 10 der 40 Transformanten (25%) überraschenderweise keine Reaktion auf die vorgenommene Schoss- und Blühinduktion mittels Vernalisation.

Es konnten von Transformanten aller drei verwendeten Konstrukte nichtschossende/nichtblühende Pflanzen erhalten werden. Die Lage der verwendeten Sequenz auf dem Gen war nicht entscheidend für die Funktion. Die erhaltenen Transformanten verhielten sich wie nicht vernalisierte Zuckerrüben. Sie begannen weder zu schossen noch zu blühen. Es wurden für jede unabhängige Transformante 30 Pflanzen getestet. Alle Pflanzen waren ohne Ausnahme schoss- und blühresistent. Keine der Pflanzen zeigte Abweichungen vom normalen Phänotyp. Die Pflanzen wurden weiter kultiviert, sie entwickelten sich weiter zu normalen Rüben mit normalem Rübenkörper. Die Schoss- und Blühresistenz hielt über den gesamten Prüfzeitraum von über 4 Monaten an (s. Fig. 2).

Ein Teil der Pflanzen wurde ein zweites Mal für nochmals 3 Monate vernalisiert. Auch nach dieser zweiten Vernalisation konnten diese Pflanzen nach Rücküberführung ins Gewächshaus bei 25 °C nicht zum Schossen oder Blühen gebracht werden.

Überraschenderweise kann somit mittels des erfindungsgemäßen transgenen Ansatzes bei Zuckerrüben die Vernalisation bzw. deren Wirkung, nämlich das Schossen und Blühen, vollständig blockiert bzw. rückgängig gemacht werden.

### Nachweis der Reprimierung von BvVIL in Zuckerrübe durch RNAi-Ansatz mittels Expressionsanalysen von Pflanzen während und nach Vernalisation.

Aus sieben Wochen alten Gewächshauspflanzen der Zuckerrübenlinien 3DC4156-wb5-1, 3DC4156-wb4-1, 3DC4156-wb4-14, 3DC4156-wb4-5, 3DC4156-wb5-18, 3DC4156-wb4-9, 3DC4156-wb4-7, 3DC4156-wb3-4, 3DC4156-wb3-4, 3DC4156-wb7-1, 3DC4156-wb18-1 und einer nicht transgenen 3DC4156, die sich die drei letzten Wochen in der Vernalisation befanden, wurde RNA mit TRI-Reagenz (Sigma, St. Louis, USA) nach dem Protokoll des Herstellers isoliert.

Die für die cDNA-Synthese eingesetzte RNA wurde per PCR auf eine Verunreinigung mit genomischer DNA überprüft, gleichzeitig wurde parallel das Ergebnis der cDNA-Synthese untersucht. Dabei wurde die FIREPol-DNA-Polymerase (Solis Biodyne, Tartu, Estland) nach den Angaben des Herstellers eingesetzt. Als Kontrolle diente Gesamt-DNA. Das PCR-Programm wurde für eine Fragmentgröße von 369 bp berechnet. Die Primer wurden von Eurofins MWG Operon (Ebersberg, Deutschland) synthetisiert und sind in Tabelle 3 aufgeführt.

**Tabelle 3: Primer zur Analyse der RNA/cDNA**

| | |
|---|---|
| vin3fwd1994 | tgacctaaatgtagtttcagttcc (SEQ ID NO: 22) |
| vin3rev2317 | tcaaagttaccgtctagagaacc (SEQ ID NO: 23) |

Gesamt-DNA wurde aus vier Wochen alten Gewächshauspflanzen der Zuckerrübenlinie 3DC4156 mit dem DNeasy-Plant-Mini-Kit (Qiagen, Hilden, Deutschland) nach dem Protokoll des Herstellers isoliert. Das RevertAid-first-strand-cDNA-Synthes-Kit von Fermentas (Vilnius, Litauen) diente zur Synthese von cDNA, wobei die Angaben des Herstellers befolgt wurden.

Die cDNA wurde als 1:10-Verdünnung für qPCR-Analysen mit einer ABI-StepOnePlus-Real-Time-PCR-Maschine verwendet, es wurde eine SYBR-green-basierende qPCR durchgeführt. Der POWER-SYBR-green-PCR-Mix von ABI (Foster City, USA) wurde als Reaktionspuffer eingesetzt. Beim Ansetzen der Reaktionsansätze wurden die Angaben des Herstellers befolgt, es wurden jeweils drei Wiederholungen durchgeführt. Die Primer für die qPCR wurden von Eurofins MWG Operon (Ebersberg, Deutschland) synthetisiert und sind in Tabelle 4 aufgeführt. Das Primerpaar nlosrealtl und nlosrealt2 diente dabei zur Normalisierung.

**Tabelle 4: Primer zur qPCR-Analyse**

| | |
|---|---|
| nlosrealt1 | GAGGAACTAGACATGGGGATACAT (SEQ ID NO: 24) |
| nlosrealt2 | GCGATACAAAGTAGACATTAGAACTC (SEQ ID NO: 25) |
| vin3realt1 | TAGGAAGCAAAGCAGGAAGGGA (SEQ ID NO: 26) |
| vin3realt2 | CCTCCGACAGAACGCATCATCT (SEQ ID NO: 27) |

Die qPCR wurde durchgeführt wie in Tabelle 5 dargestellt.

**Tabelle 5: qPCR/Programm**

| | |
|---|---|
| 95°C | 10 min |
| 95°C | 15 s |
| 58°C | 30 s |
| 72°C | 30 s |
| 95°C | 15 s |
| 60°C | 1 min |
| +0,5°C/s bis 95°C | Schmelzpunktbestimmung |

Die Schritte 2-4 wurden 40x wiederholt, Messungen fanden nach Schritt 4 und jedem Schritt der Schmelzpunktbestimmung statt.

Aus der qPCR-Analyse wurden relative Expressionsstärken für BvVIL ermittelt. Die Expressionsstärke der nicht/transgenen Kontrolle wurde dabei als 1 gesetzt. Die Ergebnisse sind in Tabelle 6 für die Pflanzen aus der Vernalisation gezeigt.

**Tabelle 6: Relative Expression von BvVIL in vernalisierten wb-Linien**

| Linie | relative Expressionsstärke |
|---|---|
| wb k 5121 | 0,154 |
| wb k 411 | 0,191 |
| wb k 4141 | 0,21 |
| wb k 451 | 0,238 |
| wb k 5181 | 0,269 |
| wb k 491 | 0,289 |
| wb k 472 | 0,384 |
| wb k 341 | 0,423 |
| wb k 716 | 0,529 |
| wb k 1811 | 0,673 |
| 3DC WT | 1 |

RNA wurde ebenfalls aus mehre Monate alten Gewächshauspflanzen der Zuckerrübenlinien 3DC4156-wb5-1, 3DC4156-wb4-1, 3DC4156-wb4-14, 3DC4156-wb4-5, 3DC4156-wb5-18, 3DC4156-wb4-9, 3DC4156-wb4-7, 3DC4156-wb3-4, 3DC4156-wb3-4, 3DC4156-wb7-1, die nach mehreren Vernalisationen nicht-schossend und -blühend blieben, und einer nicht transgenen 3DC4156, die für vier Wochen im Gewächshaus angezogen worden war, mit TRI-Reagent (Sigma, St. Louis, USA) nach dem Protokoll des Herstellers isoliert. Bei der cDNA-Synthese wurde so verfahren, wie bereits oben für die vernalisierten Pflanzen angegeben.

Für die bereits mehrmals vernalisierten, nicht schossenden und nicht blühenden Pflanzen aus dem Gewächshaus wurde eine qPCR-Analyse nach dem gleichen Muster durchgeführt. Aus dieser qPCR-Analyse wurden ebenfalls relative Expressionsstärken für BvVIL ermittelt. Die Expressionsstärke der nicht-transgenen Kontrolle wurde dabei als 1 gesetzt. Die Ergebnisse für diese Pflanzen sind in Tabelle 7 gezeigt.

**Tabelle 7: Relative Expression von BvVIL in nicht blühenden und nicht schossenden wb-Linien**

| Linie | relative Expressionsstärke |
|---|---|
| wb k 5122 | 0.11 |
| wb k 4142 | 0.15 |
| wb k 5183 | 0.17 |
| wb k 496 | 0.21 |
| wb k 412 | 0.24 |
| wb k 451 | 0.24 |
| wb k 711 | 0.35 |
| wb k 471 | 0.36 |
| wb k 342 | 0.44 |
| 3DC WT | 1 |

In beiden Ansätzen korreliert eine deutliche Reduktion des BvVIL-Transkriptes mit der Neigung zum Schossen und Blühen, und zwar derart, dass eine Reduktion des BvVIL Transkriptes auf unter 60% zu einer vollständigen Unterdrückung des Schossens und Blühens auszureichen scheint.

Eine Übersicht über die in der Anmeldung aufgeführten Sequenzen ist in der folgenden Tabelle 8 enthalten:

**Tabelle 8: Sequenzübersicht**

| SEQ ID NO: | Anmerkung |
|---|---|
| 1 | BvVIL, genomische DNA, 2366 nt |
| 2 | BvVIL, cDNA, einschließlich 3'-UTR, 2285 (3'-UTR = nt 1963-2285) |
| 3 | BvVIL, unvollständige cDNA, 1499 nt |
| 4 | BvVIL, Proteinsequenz, 653 AS |
| 5 | nt 775-1077 von SEQ ID NO 3, 303 nt |
| 6 | nt 779-1197 von SEQ ID NO 3, 419 nt |
| 7 | nt 971-1446 von SEQ ID NO 3, 476 nt |
| 8-27 | Primer |

### Sequenzprotokoll - freier Text

### Primer

Der Ausdruck "Artificial sequence" im Sequenzprotokoll bedeutet "künstliche Sequenz".

## Patentansprüche

1. Isolierte Nukleinsäure zur Inhibierung des Schossens und Blühens einer Zuckerrübenpflanze, **dadurch gekennzeichnet, dass** die Nukleinsäure eine Nukleotidsequenz umfasst, die
a) eine Sequenz der SEQ ID NO: 1-3 oder eine Teilsequenz der SEQ ID NO: 1-3, die mindestens 20 aufeinander folgende Nukleotide umfasst, aufweist, oder
b) zu einer Sequenz der SEQ ID NO: 1-3 oder zu einer Teilsequenz der SEQ ID NO: 1-3, die mindestens 20 aufeinander folgende Nukleotide umfasst, komplementär ist, oder
c) eine Sequenz der SEQ ID NO: 1-3 oder eine Teilsequenz der SEQ ID NO: 1-3, die mindestens 20 aufeinander folgende Nukleotide umfasst, oder eine dazu komplementäre Sequenz in Antisinn-Richtung aufweist, oder
d) zu einer Sequenz der SEQ ID NO: 1-3 zu mindestens 90% identisch ist oder zu einer Teilsequenz der SEQ ID NO: 1-3 mit einer Sequenzlänge von mindestens 100 Nukleotiden zu mindestens 90% identisch ist oder zu einer Teilsequenz der SEQ ID NO: 1-3 mit einer Sequenzlänge von 50-99 Nukleotiden zu mindestens 95% identisch ist, oder
e) für ein Protein mit der Aminosäuresequenz der SEQ ID NO: 4 oder einen Teil des Proteins, welches mindestens 30 aufeinander folgende Aminosäuren der SEQ ID NO: 4 umfasst, kodiert, oder
f) für ein Protein mit einer Aminosäuresequenz aus Beta vulgaris kodiert, die zu der Sequenz der SEQ ID NO: 4 zu mindestens 90% identisch oder zu einem Sequenzabschnitt, der mindestens 50 aufeinander folgende Aminosäuren der SEQ ID NO: 4 umfasst, zu mindestens 90% identisch ist, oder
g) mit einer Sequenz gemäß SEQ ID NO: 1-3 oder einer dazu komplementären Nukleotidsequenz oder einer dazu in Antisinn-Richtung orientierten Nukleotidsequenz unter stringenten Bedingungen hybridisiert und die hybridisierenden Sequenzen mindestens 90% Sequenzidentität aufweisen, wobei die stringenten Bedingungen Hybridisieren in 4 x SSC bei 65 °C und anschließendes mehrfaches Waschen in 0,1 x SSC bei 65 °C für insgesamt etwa 1 Stunde sind.

2. Nukleinsäure nach Anspruch 1, **dadurch gekennzeichnet, dass** die Teilsequenz mindestens 25, 30, 35, 40, 45, 50, 60, 70, 80, 90 oder mindestens 100 aufeinander folgende Nukleotide, besonders bevorzugt mindestens 150, 200, 250, 300, 350, 400 oder 450 aufeinander folgende Nukleotide der SEQ ID NO: 1-3 umfasst, und/oder der Teil des Proteins mindestens 40 oder 50, besonders bevorzugt mindestens 60, 70, 80, 90 oder mindestens 100 aufeinander folgende Aminosäuren der SEQ ID NO: 4, und/oder der Sequenzabschnitt mindestens 60, 70, 80 oder 90, bevorzugt mindestens 100, 120, 150, 200 oder 250 aufeinander folgende Aminosäuren der SEQ ID NO: 4, umfasst.

3. Nukleinsäure nach einem der Ansprüche 1 oder 2, **dadurch gekennzeichnet, dass** die Nukleinsäure zu mindestens 95%, 96%, 97%, 98% oder 99%, besonders bevorzugt mindestens 99,5%, 99,6%, 99,7%, 99,8% oder 99,9% zu einer Sequenz oder Teilsequenz der SEQ ID NO: 1-3 identisch ist.

4. Nukleinsäure nach einem der vorhergehenden Ansprüche, **dadurch gekennzeichnet, dass** die Nukleinsäure eine der Sequenzen der SEQ ID NO: 5, 6 oder 7, bevorzugt in Antisinnorientierung, umfasst.

5. Verwendung einer oder mehrerer Nukleinsäuren nach einem der Ansprüche 1 bis 4 zur Inhibierung des Schossens und Blühens einer Zuckerrübenpflanze.

6. Verfahren zur Herstellung einer transgenen Zuckerrübenpflanze, bei der das Schossen und Blühen nach Vernalisation inhibiert ist, umfassend die Schritte (a) des Transformierens einer Zuckerrübenzelle mit einer oder mehreren Nukleinsäuren nach einem der Ansprüche 1 bis 4 und (b) des Heranziehens einer Zuckerrübenpflanze aus der transformierten Zuckerrübenzelle.

7. Vektor oder mobiles genetisches Element, umfassend eine oder mehrere Nukleinsäuren nach einem der Ansprüche 1 bis 4.

8. Transgene Zuckerrübenpflanze, umfassend eine oder mehrere Nukleinsäuren nach einem der Ansprüche 1 bis 4 als Transgen, bei der das Schossen und Blühen inhibiert ist.

9. Samen oder Teile einer transgenen Zuckerrübenpflanze nach Anspruch 8, die mit einer oder mehreren Nukleinsäuren nach einem der Ansprüche 1 bis 4 transformiert sind und die eine oder mehreren Nukleinsäuren als Transgen enthalten.

## Claims

1. An isolated nucleic acid for inhibiting the running to seed and flowering of a sugar beet plant, **characterised in that** the nucleic acid comprises a nucleotide sequence that
a) contains or comprises a sequence of SEQ ID NO: 1-3 or a partial sequence of SEQ ID NO: 1-3 that comprises at least 20 consecutive nucleotides, or
b) is complementary to a sequence of SEQ ID NO: 1-3 or a partial sequence of SEQ ID NO: 1-3 that comprises at least 20 consecutive nucleotides, or
c) comprises a sequence of SEQ ID NO: 1-3 or a partial sequence of SEQ ID NO: 1-3 that comprises at least 20 consecutive nucleotides, or includes a complementary sequence thereto in the antisense direction, or
d) is at least 90 % identical to a sequence of SEQ ID NO: 1-3 or is at least 90 % identical to a partial sequence of SEQ ID NO: 1-3 with a sequence length of least 100 nucleotides or at least 95 % identical to a partial sequence of SEQ ID NO: 1-3 with a sequence length of 50-99 nucleotides, or
e) codes for a protein with the amino acid sequence of SEQ ID NO: 4 or a part of the protein comprising at least 30 consecutive amino acids of SEQ ID NO: 4, or
f) codes for a protein with an amino acid sequence from Beta vulgaris which is at least 90 % identical to the sequence of SEQ ID NO: 4 or at least 90 % identical to a sequence segment that comprises at least 50 consecutive amino acids of
SEQ ID NO: 4, or
g) hybridises with a sequence according to SEQ ID NO: 1-3 or a nucleotide sequence complementary thereto or a nucleotide sequence oriented in the antisense direction thereto under stringent conditions and the hybridising sequences have at least 90 % sequence identity, wherein the stringent conditions are hybridising in 4 x SSC at
65 °C and subsequent repeated washing in 0.1 x SSC at 65 °C for a total of 1 hour.

2. The nucleic acid according to claim 1, **characterised in that** the partial sequence comprises at least 25, 30, 35, 40, 45, 50, 60, 70, 80, 90 or at least 100 consecutive nucleotides, particularly preferably at least 150, 200, 250, 300, 350, 400 or 450 consecutive nucleotides of SEQ ID NO: 1-3, and/or the part of the protein comprises at least 40 or 50, particularly preferably at least 60, 70, 80, 90 or at least 100 consecutive amino acids of SEQ ID NO: 4, and/or the sequence segment comprises at least 60, 70, 80 or 90, preferably at least 100, 120, 150, 200 or 250 consecutive amino acids of SEQ ID NO: 4.

3. The nucleic acid according to either of claims 1 or 2, **characterised in that** the nucleic acid is at least 95 %, 96 %, 97 %, 98 % or 99 %, particularly preferably at least 99.5 %, 99.6 %, 99.7 %, 99.8 % or 99.9 % identical to a sequence or partial sequence of SEQ ID NO: 1-3.

4. The nucleic acid according to any one of the preceding claims, **characterised in that** the nucleic acid comprises of the sequences of SEQ ID NO: 5, 6 or 7, preferably in the antisense orientation.

5. Use of one or more nucleic acids according to any one of claims 1 to 4 for inhibiting the running to seed and flowering of a sugar beet plant.

6. A method for producing a transgenic sugar beet plant in which the running to seed and flowering is inhibited after vernalisation, comprising the steps (a) of transforming the sugar beet cell with one or more nucleic acids according to any one of claims 1 to 4 and (b) cultivating a sugar beet plant from the transformed sugar beet cell.

7. A vector or mobile genetic element comprising one or more nucleic acids according to any one of claims 1 to 4.

8. A transgenic sugar beet plant comprising one or more nucleic acids according to any one of claims 1 to 4 as the transgene, in which running to seed and flowering is inhibited.

9. Seeds or parts of a transgenic sugar beet plant according to claim 8, which have been transformed with one more more nucleic acids according to any one of claims 1 to 4 and which contain one or more nucleic acids as the transgene.

## Revendications

1. Acide nucléique isolé destiné à inhiber la pousse et la floraison d'un plan de betterave à sucre, **caractérisé en ce que** l'acide nucléique comprend une séquence de nucléotides qui
a) comprend une séquence de la SEQ ID NO: 1-3 ou une sous-séquence de la SEQ ID NO: 1-3 qui présente 20 nucléotides se succédant ou
b) est complémentaire à une séquence de la SEQ ID NO: 1-3 ou une sous-séquence de la SEQ ID NO: 1-3 qui comprend au moins 20 nucléotides se succédant ou
c) comprend une séquence de la SEQ ID NO: 1-3 ou une sous-séquence de la SEQ ID NO: 1-3 qui comprend au moins 20 nucléotides se succédant ou présente une séquence complémentaire à celle-ci en direction de l'antisens ou
d) est identique à une séquence de la SEQ ID NO: 1-3 pour au moins 90 % ou est identique à une sous-séquence de la SEQ ID NO: 1-3 ayant une longueur de séquence d'au moins 100 nucléotides pour au moins 90 % ou est identique à une sous-séquence de la SEQ ID NO: 1-3 ayant une longueur de séquence de 50 à 99 nucléotides pour au moins 95 % ou
e) code pour une protéine avec une séquence d'acide aminé de la SEQ ID NO: 4 ou une partie de la protéine qui comprend au moins 30 acides aminés se succédant de la SEQ ID NO: 4 ou
f) code pour une protéine avec une séquence d'acides aminés de beta vulgaris qui est identique à la séquence de la SEQ ID NO: 4 pour au moins 90 % ou est identique à une section de séquence qui comprend au moins 50 acides aminés se succédant de la SEQ ID NO: 4 pour au moins 90 % ou
g) s'hybride avec une séquence selon la SEQ ID NO: 1-3 ou une séquence nucléotide complémentaire à celle-ci ou une séquence nucléotide orientée en direction de l'antisens dans des conditions astringentes et les séquences hybridantes présentent au moins 90 % d'identité de séquence, les conditions astringentes étant une hybridation dans 4 x SSC à 65 °C puis un lavage à plusieurs reprises dans 0,1 x SSC à 65 °C pendant environ 1 heure au total.

2. Acide nucléique selon la revendication 1, **caractérisé en ce que** la sous-séquence comprend au moins 25, 30, 35, 40, 45, 50, 60, 70, 80, 90 ou au moins 100 nucléotides se succédant, très préférentiellement au moins 150, 200, 250, 300, 350, 400 ou 450 nucléotides se succédant de la SEQ ID NO: 1-3 et/ou la partie de la protéine comprend au moins 40 ou 50, très préférentiellement au moins 60, 70, 80, 90 ou au moins 100 acides aminés se succédant de la SEQ ID NO: 4 et/ou comprend au moins 60, 70, 80 ou 90, de préférence au moins 100, 120, 150, 200 ou 250 acides aminés se succédant de la SEQ ID NO: 4,.

3. Acide nucléique selon une des revendications 0 ou 2, **caractérisé en ce que** l'acide aminé est identique pour au moins 95 %, 96 %, 97 %, 98 % ou 99 %, très préférentiellement au moins 99,5 %, 99,6 %, 99,7 %, 99,8 % ou 99,9 %, à une séquence ou sous-séquence de la SEQ ID NO: 1-3.

4. Acide nucléique selon une des revendications précédentes, **caractérisé en ce que** l'acide aminé comprend une des séquences de la SEQ ID NO: 5, 6 ou 7, de préférence en orientation antisens.

5. Utilisation d'un ou plusieurs acides nucléiques selon une des revendications 0 à 4 pour inhiber la pousse et la floraison d'un plan de betterave à sucre.

6. Procédé de production d'un plan de betterave à sucre transgénique dans lequel la pousse et la floraison sont inhibées après vernalisation, comprenant les étapes (a) de transformation d'une cellule de betterave à sucre avec un ou plusieurs acides nucléiques selon une des revendications 0 à 4 et (b) de création d'un plan de betterave à sucre à partir de la cellule de betterave à sucre transformée.

7. Vecteur ou élément génétique mobile, comprenant un ou plusieurs acides nucléiques selon une des revendications 0 à 4.

8. Plan de betterave à sucre transgénique, comprenant un ou plusieurs acides nucléiques selon une des revendications 0 à 4 sous forme de transgène, dans lequel la pousse et la floraison sont inhibées.

9. Graines ou parties d'un plan de betterave à sucre transgénique selon la revendication 8, qui sont transformées avec un ou plusieurs acides nucléiques selon une des revendications 1 à 4 et qui contiennent un ou plusieurs acides nucléiques faisant office de transgène.


### SEQUENZPROTOKOLL

<110> KWS SAAT AG
<120> Inhibierung des Schossens und Blühens einer Zuckerrübenpflanze
<130> KWS0168PCT
<150> 102009041333.2
   <151> 2009-09-15
<160> 27
<170> PatentIn version 3.5
<210> 1
   <211> 2366
   <212> DNA
   <213> Beta vulgaris
<400> 1
   atgatagaac cgcagctgaa agcatgcaac aaaaatgtga agaatccgga gagcaggaag      60
   actgcttcca cttcgtacaa ttctgcttct aggaagcaaa gcaggaaggg agaaaatcct     120
   attcgtgtta ctccgttagg agagcaatct tctgattttg gatgttctag tacttggata     180
   tgtaaaaatt ctgcatgtag agctgttctg tctatagatg atgcgttctg tcggaggtgt     240
   tcatgctgca tctgtcatca atttgatgat aataaagacc ctagtctttg gttggtttgt     300
   gaatccgagt ctgggcaggg tgattcttgt ggattatcat gccatattga gtgtgcattt     360
   caacaagaaa agctgggagt tgtgaacctt gggcaataca tgcatttgga tgggagttac     420
   tgttgttctt cttgcggcaa agtctctggg atacttgggt cagtacttct gttttatgta     480
   gtgagatatt gacctgaagt catgcttgtt ttgatggaag ataaataatt taaaaaaaat     540
   gttacatgcc caactattac aaggccagta gaaaactatg agatatatta attttgatat     600
   tactgtggag gctcagttga atttatatgc ttgagtttta ctcactaatg gcaggtgttg     660
   gaaaaagcaa ttggctatag ctaaggatgc tcgacgtgtc gatgtgcttt gctatagaat     720
   atttttgagt tacagactcc tcgagggcac agctaagttt aaggacctcc acgagattgt     780
   tgcagaagct aaaacaaagc tggaggcaga ggtgggtcct atgaacggag actctgtcaa     840
   gatggccaga ggtattgtta gcaggcttgc tattgctgca gatgtgcaaa agctctgttc     900
   gcacgcgatt gataaagcta atgaatggct cgccaatgtt tctagcatta gttcaaattg     960
   caaaggttag aatacataca gcctttattg ttctccattt acttggtgag tattctatta    1020
   taataaatta ttaatttctt ttggcataat ggtggcagtg gatgcacttc ctgctgcatg    1080
   caggtttcta tttgaagaag ttacttcttg ttcacttgct atagttttga tagatatccc     1140
   cacaccaatg actgattccg tcaaaggcta caagctatgg tactgcaaaa gtagacatga     1200
   gacttttgca agggagccta catccgtctt tccaagggag aaaagaaaaa tatctgtaaa     1260
   gaatctcaag ccttgcaccg agtacacatt cagaatagtt tcctacacag aagttggtga     1320
   tttaggccac tctgaggcta agtgtttcac caagagtttg gagatcatta gtaagaaatc     1380
   caccacagtg ggctgtaaga aggaagatcc ttgtgttgag aggagctcct cgaatgcaaa     1440
   ggaacaacat aattcaaatt tggctgcaat atcttctgga ttcaaggtgc gggaccttgg     1500
   gaaaatcttg cacctagcat gggcccaaga acagggttgc cttgaaggtt tctgcagtgc     1560
   tgatgtagaa caatgctgtg gagtaactaa atgtgaatct ccaaaagatc accagtcacc     1620
   tccacctgtt tctcgtgagc ttgacctaaa tgtagtttca gttcctgatt taaatgaaga     1680
   ccttacccct cccttagagt cttcaaggga tgaagacaac ggatgcacgc tagagcgtgc     1740
   tactgggcct gatgatgatg ctgcttccca tggtgttgag aagaatgggc ttgggctagc     1800
   caggtcaaat ggtagtgggc caagtgatga gtctcaagct tgggctctca tccgaaatgg     1860
   agatgtgcct gctgttgatt ccttggcaga gacccgtcgg aagaggtctt caagtgcgaa     1920
   tgaagaaaca catgactgtg acagcactct gataaatgga tcgccatttc ggatttcagg     1980
   cgggcctggt tctctagacg gtaactttga gtattgtgtg aaggtcatcc ggtggttgga     2040
   gtgtgagggc tatctaaaac aggaatttag attgaaatta ttgacttggt ttagcttaag     2100
   atctactgaa caagagcgtc gggtagtcag cactttcatt caaactctga tggatgatcc     2160
   aaagagctta gcaggacagc tagttgattc ctttggagat ctcatatcca gcaagaggcc     2220
   caggactagt ttcactagta agtttatgtc tatagttgtt ctttgattga gaaattttat     2280
   gtcttctttc aaacatttct attactcttc ttattctgaa gttttggact aattttgtca     2340
   tgctaattac aggcattcct tcctaa                                          2366
<210> 2
   <211> 2285
   <212> DNA
   <213> Beta vulgaris
<220>
   <221> 3'UTR
   <222> (1963)..(2285)
<400> 2
   atgatagaac cgcagctgaa agcatgcaac aaaaatgtga agaatccgga gagcaggaag       60
   actgcttcca cttcgtacaa ttctgcttct aggaagcaaa gcaggaaggg agaaaatcct      120
   attcgtgtta ctccgttagg agagcaatct tctgattttg gatgttctag tacttggata      180
   tgtaaaaatt ctgcatgtag agctgttctg tctatagatg atgcgttctg tcggaggtgt      240
   tcatgctgca tctgtcatca atttgatgat aataaagacc ctagtctttg gttggtttgt      300
   gaatccgagt ctgggcaggg tgattcttgt ggattatcat gccatattga gtgtgcattt      360
   caacaagaaa agctgggagt tgtgaacctt gggcaataca tgcatttgga tgggagttac      420
   tgttgttctt cttgcggcaa agtctctggg atacttgggt gttggaaaaa gcaattggct      480
   atagctaagg atgctcgacg tgtcgatgtg ctttgctata gaatattttt gagttacaga      540
   ctcctcgagg gcacagctaa gtttaaggac ctccacgaga ttgttgcaga agctaaaaca      600
   aagctggagg cagaggtggg tcctatgaac ggagactctg tcaagatggc cagaggtatt      660
   gttagcaggc ttgctattgc tgcagatgtg caaaagctct gttcgcacgc gattgataaa      720
   gctaatgaat ggctcgccaa tgtttctagc attagttcaa attgcaaagt ggatgcactt      780
   cctgctgcat gcaggtttct atttgaagaa gttacttctt gttcacttgc tatagttttg      840
   atagatatcc ccacaccaat gactgattcc gtcaaaggct acaagctatg gtactgcaaa      900
   agtagacatg agacttttgc aagggagcct acatccgtct ttccaaggga gaaaagaaaa      960
   atatctgtaa agaatctcaa gccttgcacc gagtacacat tcagaatagt ttcctacaca     1020
   gaagttggtg atttaggcca ctctgaggct aagtgtttca ccaagagttt ggagatcatt     1080
   agtaagaaat ccaccacagt gggctgtaag aaggaagatc cttgtgttga gaggagctcc     1140
   tcgaatgcaa aggaacaaca taattcaaat ttggctgcaa tatcttctgg attcaaggtg     1200
   cgggaccttg ggaaaatctt gcacctagca tgggcccaag aacagggttg ccttgaaggt     1260
   ttctgcagtg ctgatgtaga acaatgctgt ggagtaacta aatgtgaatc tccaaaagat     1320
   caccagtcac ctccacctgt ttctcgtgag cttgacctaa atgtagtttc agttcctgat     1380
   ttaaatgaag accttacccc tcccttagag tcttcaaggg atgaagacaa cggatgcacg     1440
   ctagagcgtg ctactgggcc tgatgatgat gctgcttccc atggtgttga gaagaatggg     1500
   cttgggctag ccaggtcaaa tggtagtggg ccaagtgatg agtctcaagc ttgggctctc     1560
   atccgaaatg gagatgtgcc tgctgttgat tccttggcag agacccgtcg gaagaggtct     1620
   tcaagtgcga atgaagaaac acatgactgt gacagcactc tgataaatgg atcgccattt     1680
   cggatttcag gcgggcctgg ttctctagac ggtaactttg agtattgtgt gaaggtcatc     1740
   cggtggttgg agtgtgaggg ctatctaaaa caggaattta gattgaaatt attgacttgg     1800
   tttagcttaa gatctactga acaagagcgt cgggtagtca gcactttcat tcaaactctg     1860
   atggatgatc caaagagctt agcaggacag ctagttgatt cctttggaga tctcatatcc     1920
   agcaagaggc ccaggactag tttcactagc attccttcct aaataaatct taactaagga     1980
   cggcacacat atcttggata caattcagat gtttaggaca caatttttag gaggcagtac     2040
   ctgattttcc tcgagaaagg gattccatca gtggttaact gcacatttta gaaggtattt     2100
   gttagagttt ccttgaccac atttgtagaa agattcacat tgagacaatc attgttgcct     2160
   tctcgcattg aaggaaggat atatgcttca atgaatattt aaattctagt tcaatttact     2220
   aattaattag tttgttttct caaaaaaaaa aaaaaaaaaa aaaaagtact agtcgacgcg     2280
   tggcc                                                                2285
<210> 3
   <211> 1499
   <212> DNA
   <213> Beta vulgaris
<400> 3
   agaggaggaa aaaggaggaa tgactataga agaatatcta agcgatgcat tgttagatgg      60
   atggatatct gttggagttg aaaactggct gtttagagca tgagtttgga agatcaaatc     120
   cctacaatag gtattcaaaa atctttatta gtgtgcaaac tgcaaaggac cgcggagaaa     180
   aatggaaatc ccagttatac tttgagatgg ttagaacttt tctggagaat tcctgaaatc     240
   tgttccaaac tccaaagaga gcgatcaaga cttttttttt ttttttttga caaagaaaat     300
   aaaaattaga atcctctctg taaggcagta tgatagaacc gcagctgaaa gcatgcaaca     360
   aaaatgtgaa gaatccggag agcaggaaga ctgcttccac ttcgtacaat tctgcttcta     420
   ggaagcaaag caggaaggga gaaaatccta ttcgtgttac tccgttagga gagcaatctt     480
   ctgattttgg atgttctagt acttggatat gtaaaaattc tgcatgtaga gctgttctgt     540
   ctatagatga tgcgttctgt cggaggtgtt catgctgcat ctgtcatcaa tttgatgata     600
   ataaagaccc tagtctttgg ttggtttgtg aatccgagtc tgggcagggt gattcttgtg     660
   gattatcatg ccatattgag tgtgcatttc aacaagaaaa gctgggagtt gtgaaccttg     720
   ggcaatacat gcatttggat gggagttact gttgttcttc ttgcggcaaa gtctctggga     780
   tacttgggtg ttggaaaaag caattggcta tagctaagga tgctcgacgt gtcgatgtgc     840
   tttgctatag aatatttttg agttacagac tcctcgaggg cacagctaag tttaaggacc     900
   tccacgagat tgttgcagaa gctaaaacaa agctggaggc agaggtgggt cctatgaacg     960
   gagactctgt caagatggcc agaggtattg ttagcaggct tgctattgct gcagatgtgc    1020
   aaaagctctg ttcgcacgcg attgataaag ctaatgaatg gctcgccaat gtttctagca    1080
   ttagttcaaa ttgcaaagtg gatgcacttc ctgctgcatg caggtttcta tttgaagaag    1140
   ttacttcttg ttcacttgct atagttttga tagatatccc cacaccaatg actgattccg    1200
   tcaaaggcta caagctatgg tactgcaaaa gtagacatga gacttttgca agggagccta    1260
   catccgtctt tccaagggag aaaagaaaaa tatctgtaaa gaatctcaag ccttgcaccg    1320
   agtacacatt cagaatagtt tcctacacag aagttggtga tttaggccac tctgaggcta    1380
   agtgtttcac caagagtttg gagatcatta gtaagaaatc caccacagtg ggctgtaaaa    1440
   aggaagatcc ttgtgttgag aggagctcct cgaatgcaag agaacacata attcaaatt     1499
<210> 4
   <211> 653
   <212> PRT
   <213> Beta vulgaris
<400> 4
   Met Ile Glu Pro Gln Leu Lys Ala Cys Asn Lys Asn Val Lys Asn Pro
   1               5                   10                  15
   Glu Ser Arg Lys Thr Ala Ser Thr Ser Tyr Asn Ser Ala Ser Arg Lys
               20                  25                  30
   Gln Ser Arg Lys Gly Glu Asn Pro Ile Arg Val Thr Pro Leu Gly Glu
           35                  40                  45
   Gln Ser Ser Asp Phe Gly Cys Ser Ser Thr Trp Ile Cys Lys Asn Ser
       50                  55                  60
   Ala Cys Arg Ala Val Leu Ser Ile Asp Asp Ala Phe Cys Arg Arg Cys
   65                  70                  75                  80
   Ser Cys Cys Ile Cys His Gln Phe Asp Asp Asn Lys Asp Pro Ser Leu
                   85                  90                  95
   Trp Leu Val Cys Glu Ser Glu Ser Gly Gln Gly Asp Ser Cys Gly Leu
               100                 105                 110
   Ser Cys His Ile Glu Cys Ala Phe Gln Gln Glu Lys Leu Gly Val Val
           115                 120                 125
   Asn Leu Gly Gln Tyr Met His Leu Asp Gly Ser Tyr Cys Cys Ser Ser
       130                 135                 140
   Cys Gly Lys Val Ser Gly Ile Leu Gly Cys Trp Lys Lys Gln Leu Ala
   145                 150                 155                 160
   Ile Ala Lys Asp Ala Arg Arg Val Asp Val Leu Cys Tyr Arg Ile Phe
                   165                 170                 175
   Leu Ser Tyr Arg Leu Leu Glu Gly Thr Ala Lys Phe Lys Asp Leu His
               180                 185                 190
   Glu Ile Val Ala Glu Ala Lys Thr Lys Leu Glu Ala Glu Val Gly Pro
           195                 200                 205
   Met Asn Gly Asp Ser Val Lys Met Ala Arg Gly Ile Val Ser Arg Leu
   210 215 220
   Ala Ile Ala Ala Asp Val Gln Lys Leu Cys Ser His Ala Ile Asp Lys
   225 230 235 240
   Ala Asn Glu Trp Leu Ala Asn Val Ser Ser Ile Ser Ser Asn Cys Lys
   245 250 255
   Val Asp Ala Leu Pro Ala Ala Cys Arg Phe Leu Phe Glu Glu Val Thr
   260 265 270
   Ser Cys Ser Leu Ala Ile Val Leu Ile Asp Ile Pro Thr Pro Met Thr
   275 280 285
   Asp Ser Val Lys Gly Tyr Lys Leu Trp Tyr Cys Lys Ser Arg His Glu
   290 295 300
   Thr Phe Ala Arg Glu Pro Thr Ser Val Phe Pro Arg Glu Lys Arg Lys
   305 310 315 320
   Ile Ser Val Lys Asn Leu Lys Pro Cys Thr Glu Tyr Thr Phe Arg Ile
   325 330 335
   Val Ser Tyr Thr Glu Val Gly Asp Leu Gly His Ser Glu Ala Lys Cys
   340 345 350
   Phe Thr Lys Ser Leu Glu Ile Ile Ser Lys Lys Ser Thr Thr Val Gly
   355 360 365
   Cys Lys Lys Glu Asp Pro Cys Val Glu Arg Ser Ser Ser Asn Ala Lys
   370 375 380
   Glu Gln His Asn Ser Asn Leu Ala Ala Ile Ser Ser Gly Phe Lys Val
   385 390 395 400
   Arg Asp Leu Gly Lys Ile Leu His Leu Ala Trp Ala Gln Glu Gln Gly
   405 410 415
   Cys Leu Glu Gly Phe Cys Ser Ala Asp Val Glu Gln Cys Cys Gly Val
   420 425 430
   Thr Lys Cys Glu Ser Pro Lys Asp His Gln Ser Pro Pro Pro Val Ser
   435 440 445
   Arg Glu Leu Asp Leu Asn Val Val Ser Val Pro Asp Leu Asn Glu Asp
   450 455 460
   Leu Thr Pro Pro Leu Glu Ser Ser Arg Asp Glu Asp Asn Gly Cys Thr
   465 470 475 480
   Leu Glu Arg Ala Thr Gly Pro Asp Asp Asp Ala Ala Ser His Gly Val
   485 490 495
   Glu Lys Asn Gly Leu Gly Leu Ala Arg Ser Asn Gly Ser Gly Pro Ser
   500 505 510
   Asp Glu Ser Gln Ala Trp Ala Leu Ile Arg Asn Gly Asp Val Pro Ala
   515 520 525
   Val Asp Ser Leu Ala Glu Thr Arg Arg Lys Arg Ser Ser Ser Ala Asn
   530 535 540
   Glu Glu Thr His Asp Cys Asp Ser Thr Leu Ile Asn Gly Ser Pro Phe
   545 550 555 560
   Arg Ile Ser Gly Gly Pro Gly Ser Leu Asp Gly Asn Phe Glu Tyr Cys
   565 570 575
   Val Lys Val Ile Arg Trp Leu Glu Cys Glu Gly Tyr Leu Lys Gln Glu
   580 585 590
   Phe Arg Leu Lys Leu Leu Thr Trp Phe Ser Leu Arg Ser Thr Glu Gln
   595 600 605
   Glu Arg Arg Val Val Ser Thr Phe Ile Gln Thr Leu Met Asp Asp Pro
   610 615 620
   Lys Ser Leu Ala Gly Gln Leu Val Asp Ser Phe Gly Asp Leu Ile Ser
   625 630 635 640
   Ser Lys Arg Pro Arg Thr Ser Phe Thr Ser Ile Pro Ser
   645 650
<210> 5
   <211> 303
   <212> DNA
   <213> Beta vulgaris
<400> 5
   ctgggatact tgggtgttgg aaaaagcaat tggctatagc taaggatgct cgacgtgtcg      60
   atgtgctttg ctatagaata tttttgagtt acagactcct cgagggcaca gctaagttta     120
   aggacctcca cgagattgtt gcagaagcta aaacaaagct ggaggcagag gtgggtccta     180
   tgaacggaga ctctgtcaag atggccagag gtattgttag caggcttgct attgctgcag     240
   atgtgcaaaa gctctgttcg cacgcgattg ataaagctaa tgaatggctc gccaatgttt     300
   cta                                                                   303
<210> 6
   <211> 419
   <212> DNA
   <213> Beta vulgaris
<400> 6
   gatacttggg tgttggaaaa agcaattggc tatagctaag gatgctcgac gtgtcgatgt      60
   gctttgctat agaatatttt tgagttacag actcctcgag ggcacagcta agtttaagga     120
   cctccacgag attgttgcag aagctaaaac aaagctggag gcagaggtgg gtcctatgaa     180
   cggagactct gtcaagatgg ccagaggtat tgttagcagg cttgctattg ctgcagatgt     240
   gcaaaagctc tgttcgcacg cgattgataa agctaatgaa tggctcgcca atgtttctag     300
   cattagttca aattgcaaag tggatgcact tcctgctgca tgcaggtttc tatttgaaga     360
   agttacttct tgttcacttg ctatagtttt gatagatatc cccacaccaa tgactgatt      419
<210> 7
   <211> 476
   <212> DNA
   <213> Beta vulgaris
<400> 7
   caagatggcc agaggtattg ttagcaggct tgctattgct gcagatgtgc aaaagctctg 60
   ttcgcacgcg attgataaag ctaatgaatg gctcgccaat gtttctagca ttagttcaaa 120
   ttgcaaagtg gatgcacttc ctgctgcatg caggtttcta tttgaagaag ttacttcttg 180
   ttcacttgct atagttttga tagatatccc cacaccaatg actgattccg tcaaaggcta 240
   caagctatgg tactgcaaaa gtagacatga gacttttgca agggagccta catccgtctt 300
   tccaagggag aaaagaaaaa tatctgtaaa gaatctcaag ccttgcaccg agtacacatt 360
   cagaatagtt tcctacacag aagttggtga tttaggccac tctgaggcta agtgtttcac 420
   caagagtttg gagatcatta gtaagaaatc caccacagtg ggctgtaaaa aggaag 476
<210> 8
   <211> 27
   <212> DNA
   <213> Artificial Sequence
<220>
   <223> Primer
<400> 8
   ctgggatact tgggtgttgg aaaaagc 27
<210> 9
   <211> 28
   <212> DNA
   <213> Artificial Sequence
<220>
   <223> Primer
<400> 9
   tagaaacatt ggcgagccat tcattagc 28
<210> 10
   <211> 24
   <212> DNA
   <213> Artificial Sequence
<220>
   <223> Primer
<400> 10
   gatacttggg tgttggaaaa agca 24
<210> 11
   <211> 23
   <212> DNA
   <213> Artificial Sequence
<220>
   <223> Primer
<400> 11
   aatcagtcat tggtgtgggg ata 23
<210> 12
   <211> 21
   <212> DNA
   <213> Artificial Sequence
<220>
   <223> Primer
<400> 12
   caagatggcc agaggtattg t 21
<210> 13
   <211> 21
   <212> DNA
   <213> Artificial Sequence
<220>
   <223> Primer
<400> 13
   cttccttttt acägcccact g 21
<210> 14
   <211> 30
   <212> DNA
   <213> Artificial Sequence
<220>
   <223> Primer
<400> 14
   gagaggacgt cgacctggga tacttgggtg 30
<210> 15
   <211> 26
   <212> DNA
   <213> Artificial Sequence
<220>
   <223> Primer
<400> 15
   ccccccgggt agaaacattg gcgagc 26
<210> 16
   <211> 30
   <212> DNA
   <213> Artificial Sequence
<220>
   <223> Primer
<400> 16
   gagaggacgt cgacgatact tgggtgttgg 30
<210> 17
   <211> 32
   <212> DNA
   <213> Artificial Sequence
<220>
   <223> Primer
<400> 17
   ccccccggga atcagtcatt ggtgtgggga ta 32
<210> 18
   <211> 30
   <212> DNA
   <213> Artificial Sequence
<220>
   <223> Primer
<400> 18
   gagaggacct cgagcaagat ggccagaggt 30
<210> 19
   <211> 25
   <212> DNA
   <213> Artificial Sequence
<220>
   <223> Primer
<400> 19
   gtcgaccccc ccgggcttcc ttttt 25
<210> 20
   <211> 19
   <212> DNA
   <213> Artificial Sequence
<220>
   <223> Primer
<400> 20
   gtggagaggc tattcggta 19
<210> 21
   <211> 20
   <212> DNA
   <213> Artificial Sequence
<220>
   <223> Primer
<400> 21
   ccaccatgat attcggcaag 20
<210> 22
   <211> 24
   <212> DNA
   <213> Artificial Sequence
<220>
   <223> Primer
<400> 22
   tgacctaaat gtagtttcag ttcc 24
<210> 23
   <211> 23
   <212> DNA
   <213> Artificial Sequence
<220>
   <223> Primer
<400> 23
   tcaaagttac cgtctagaga acc 23
<210> 24
   <211> 24
   <212> DNA
   <213> Artificial Sequence
<220>
   <223> Primer
<400> 24
   gaggaactag acatggggat acat 24
<210> 25
   <211> 26
   <212> DNA
   <213> Artificial Sequence
<220>
   <223> Primer
<400> 25
   gcgatacaaa gtagacattagaactc 26
<210> 26
   <211> 22
   <212> DNA
   <213> Artificial Sequence
<220>
   <223> Primer
<400> 26
   taggaagcaa agcaggaagg ga 22
<210> 27
   <211> 22
   <212> DNA
   <213> Artificial Sequence
<220>
   <223> Primer
<400> 27
   cctccgacag aacgcatcat ct 22